(19) Europäisches Patentamt
European Patent Office
Office européen des brevets

(11) EP 4 692 063 A1

(12) **EUROPEAN PATENT APPLICATION**
published in accordance with Art. 153(4) EPC

(43) Date of publication:
**11.02.2026 Bulletin 2026/07**

(21) Application number: **24778136.2**

(22) Date of filing: **28.03.2024**

(51) International Patent Classification (IPC):
***C07D 221/20*** (2006.01) ***C07D 471/10*** (2006.01)
***C07D 209/54*** (2006.01) ***C07C 69/734*** (2006.01)
***A61P 7/00*** (2006.01) ***A61P 9/00*** (2006.01)
***A61P 37/02*** (2006.01) ***A61P 25/28*** (2006.01)
***A61P 11/00*** (2006.01) ***A61P 13/00*** (2006.01)
***A61P 13/12*** (2006.01) ***A61P 27/02*** (2006.01)
***A61P 29/00*** (2006.01) ***A61P 19/02*** (2006.01)
***A61P 17/06*** (2006.01) ***A61P 21/04*** (2006.01)
***A61P 31/14*** (2006.01) ***A61K 31/438*** (2006.01)
***A61K 31/395*** (2006.01)

(52) Cooperative Patent Classification (CPC):
**A61K 31/395; A61K 31/438; A61P 7/00; A61P 9/00; A61P 11/00; A61P 13/00; A61P 13/12; A61P 17/06; A61P 19/02; A61P 21/04; A61P 25/28; A61P 27/02; A61P 29/00; A61P 31/14; A61P 37/02;**
(Cont.)

(86) International application number:
**PCT/CN2024/084387**

(87) International publication number:
**WO 2024/199350 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.03.2023 CN 202310317527**
**29.02.2024 CN 202410227541**

(71) Applicant: **Wuhan Createrna Science and Technology Co.,Ltd.**
**Wuhan, Hubei 430075 (CN)**

(72) Inventors:
- **HU, Qinquan**
  **Wuhan, Hubei 430075 (CN)**
- **LOU, Jun**
  **Wuhan, Hubei 430075 (CN)**
- **WANG, Liang**
  **Wuhan, Hubei 430075 (CN)**
- **ZHANG, Yihan**
  **Wuhan, Hubei 430075 (CN)**

(74) Representative: **Michalski Hüttermann & Partner Patentanwälte mbB**
**Kaistraße 16A**
**40221 Düsseldorf (DE)**

# (54) CRYSTAL FORMS OF NITROGEN-CONTAINING SPIRO COMPOUND, PREPARATION METHOD THEREFOR AND USE THEREOF

(57) Disclosed in the present invention are crystal forms of a nitrogen-containing spiro compound, a preparation method therefor and the use thereof. Provided in the present invention is a crystal form I of a nitrogen-containing spiro compound as shown in formula A, which crystal form I has an X-ray powder diffraction pattern comprising diffraction peaks at 5.67±0.20°, 11.37 ±0.20°, 16.69±0.20°, 17.32±0.20° and 19.73±0.20° 2θ, as determined by using Cu-Kα radiation. Provided in the present invention is a crystal form II of the nitrogen-containing spiro compound as shown in formula A, which crystal form II has an X-ray powder diffraction pattern comprising diffraction peaks at 11.32±0.20°, 11.70 ±0.20°, 11.93±0.20°, 18.03±0.20°, 18.81±0.20° and 19.17±0.20° 2θ, as determined by using Cu-Kα radiation. The crystal forms of the nitrogen-containing spiro compound of the present invention have good stability, an inhibitory activity on complement factor D, a good inhibitory effect on rabbit erythrocyte hemolysis, and relatively high in-vivo exposure and relatively high oral bioa-



Processed by Luminess, 75001 PARIS (FR)

(Cont. next page)

vailability.

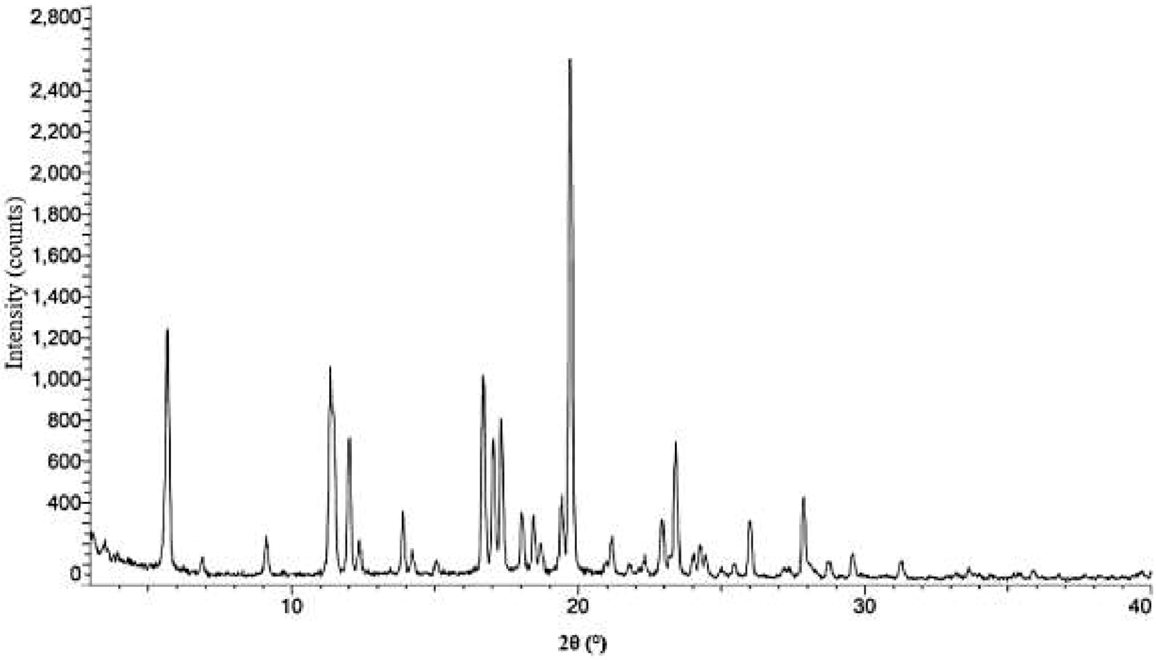


FIG. 2

(52) Cooperative Patent Classification (CPC): (Cont.)
**C07C 69/734; C07D 209/54; C07D 221/20; C07D 471/10**

## Description

SPECIFICATION

The present application claims the priority of Chinese Patent Application No. 2023103175276, filed on March 28, 2023, and Chinese Patent Application No. 2024102275411, filed on February 29, 2024. The contents of the above Chinese patent applications are incorporated herein by reference in their entirety.

TECHNICAL FIELD

The present disclosure relates to crystal forms of a nitrogen-containing spiro compound, a preparation method therefor, and a use thereof.

BACKGROUND

Complement is a protein widely present in the serum, tissue fluid, and cell membrane surfaces of humans and vertebrates. It mediates immune and inflammatory responses, mostly existing as glycoproteins, and is produced by various cells such as hepatocytes, macrophages, and intestinal mucosal epithelial cells. Complement is the necessary supplementary condition for antibodies to achieve their cytolytic effect, hence its name, but it actually mediates both specific and non-specific immunity. The three activation pathways of the complement system include the classical pathway, the mannan-binding lectin (MBL) pathway, and the alternative (bypass) pathway. Complement factor D plays an early and central role in the cascade activation of the alternative complement pathway. Activation of the alternative complement pathway is initiated by the spontaneous hydrolysis of the thioester bond within C3, generating C3($H_2O$), which associates with factor B to form the C3($H_2O$)B complex. The role of complement factor D is to cleave factor B within the C3($H_2O$)B complex to form Ba and Bb. In addition to binding with C3b to form the C3 convertase, Bb is involved in the proliferation of pre-activated B lymphocytes, whereas Ba inhibits their proliferation. Factor D is highly expressed in adipose tissue, stimulates glucose transport, promotes triglyceride accumulation in adipocytes, and inhibits lipolysis.

Dysregulation of the complement system plays an important role in the pathogenesis of IgA nephropathy (IgAN), lupus nephritis (LN), and paroxysmal nocturnal hemoglobinuria (PNH), and the deposition of complement components and immune complex is often found in the renal pathological tests of IgAN and LN. Complement is the direct cause of hemolysis in PNH, and C5aR is involved in the amplification of complement system damage. CFB and CFD are key components of the complement bypass pathway and are directly involved in the regulation of complement activation. Therefore, C5aR, CFB, and CFD are closely related to the pathogenesis of IgAN, LN, and PNH.

Paroxysmal nocturnal hemoglobinuria (PNH) is a rare and life-threatening blood disease, characterized by complement-driven hemolysis, thrombosis, and impaired bone marrow function, leading to anemia, fatigue, and other debilitating symptoms, which will seriously affect the quality of life of patients. The drugs currently on the market for PNH are mainly monoclonal antibody drugs Soliris and Ultomiris. Among them, Soliris was first approved for marketing in 2007 and has been approved for a variety of super rare diseases, including paroxysmal nocturnal hemoglobinuria (PNH), atypical hemolytic uremic syndrome (aHUS), generalized myasthenia gravis (gMG), and neuromyelitis optica spectrum disorder (NMOSD). Ultomiris is an upgraded product of Soliris, a second-generation and long-acting C5 complement inhibitor, which was first approved for marketing at the end of 2018. The approved indications include: PNH and aHUS. Despite treatment with current anti-C5 standard-of-care therapies, a large proportion of PNH patients remain anemic and transfusion dependent.

At present, there are no drugs on the market for small molecule inhibitors of complement factor D targeting the complement alternative pathway, which is a key driver of complement-driven renal disease (CDRD). Therefore, it is of positive significance to develop small-molecule inhibitors with good biological activity for the treatment of the above diseases. Simultaneously, developing pharmaceutical forms of these compounds suitable for drug use has become an urgent technical problem to be addressed.

SUMMARY

The technical problem addressed by the present disclosure is the limitation of the variety and crystal forms of existing complement factor D inhibitors. Accordingly, the present disclosure provides a crystal form of a nitrogen-containing spiro compound, a preparation method thereof, and a use thereof. The crystal forms of the nitrogen-containing spiro compound of the present invention have good stability, an inhibitory activity on complement factor D, a good inhibitory effect on rabbit erythrocyte hemolysis, and relatively high *in vivo* exposure and relatively high oral bioavailability.

Provided in the present disclosure is a crystal form I of a nitrogen-containing spiro compound of formula A, wherein the crystal form I has an X-ray powder diffraction (XRPD) pattern comprising diffraction peaks at 5.67±0.20°,

11.37±0.20°, 16.69±0.20°, 17.32±0.20°, and 19.73±0.20°, as determined by using Cu-Kα radiation and expressed in terms of 2θ angles;

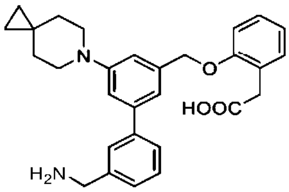

A

.

The X-ray powder diffraction (XRPD) pattern of the crystal form I, as determined by using Cu-Kα radiation and expressed in terms of 2θ angles, may further comprise diffraction peaks at one or more of the following positions: 12.01 ±0.20°, 13.89±0.20°, 17.05±0.20°, 19.42±0.20°, 23.41±0.20°, and 27.86±0.20°.

The X-ray powder diffraction (XRPD) pattern of the crystal form I, as determined by using Cu-Kα radiation and expressed in terms of 2θ angles, may further comprise diffraction peaks at one or more of the following positions: 18.04 ±0.20°, 18.43±0.20°, 22.94±0.20°, and 26.01±0.20°.

The X-ray powder diffraction (XRPD) pattern of the crystal form I, as determined by using Cu-Kα radiation and expressed in terms of 2θ angles, may further comprise diffraction peaks at 5.67±0.20°, 11.37±0.20°, 12.01±0.20°, 13.89 ±0.20°, 16.69±0.20°, 17.05±0.20°, 17.32±0.20°, 18.04±0.20°, 18.43±0.20°, 19.42±0.20°, 19.73±0.20°, 22.94 ±0.20°, 23.41±0.20°, 26.01±0.20°, and 27.86±0.20°.

The X-ray powder diffraction (XRPD) pattern of the crystal form I, as determined by using Cu-Kα radiation and expressed in terms of 2θ angles, may comprise diffraction peaks at positions as shown in the following table:

| No. | Position in 2θ (°) | No. | Position in 2θ (°) |
|---|---|---|---|
| 1 | 3.50 | 23 | 22.94 |
| 2 | 5.67 | 24 | 23.41 |
| 3 | 6.88 | 25 | 24.03 |
| 4 | 9.13 | 26 | 24.23 |
| 5 | 11.37 | 27 | 24.45 |
| 6 | 12.01 | 28 | 25.00 |
| 7 | 12.37 | 29 | 25.45 |
| 8 | 13.89 | 30 | 26.01 |
| 9 | 14.20 | 31 | 27.19 |
| 10 | 15.05 | 32 | 27.36 |
| 11 | 16.69 | 33 | 27.86 |
| 12 | 17.05 | 34 | 28.76 |
| 13 | 17.32 | 35 | 29.57 |
| 14 | 18.04 | 36 | 31.29 |
| 15 | 18.43 | 37 | 33.18 |
| 16 | 18.69 | 38 | 33.62 |
| 17 | 19.42 | 39 | 35.40 |
| 18 | 19.73 | 40 | 35.87 |
| 19 | 20.98 | 41 | 36.75 |
| 20 | 21.17 | 42 | 38.23 |
| 21 | 21.80 | 43 | 39.71 |
| 22 | 22.32 | | |

The X-ray powder diffraction (XRPD) pattern of the crystal form I, as determined by using Cu-Kα radiation and expressed in terms of 2θ angles, may comprise diffraction peaks at positions and relative intensities as shown in the following table:

| No. | Position in 2θ (°) | Relative intensity | No. | Position in 2θ (°) | Relative intensity |
|---|---|---|---|---|---|
| 1 | 3.50 | 3.00% | 23 | 22.94 | 10.60% |
| 2 | 5.67 | 45.80% | 24 | 23.41 | 25.50% |
| 3 | 6.88 | 3.00% | 25 | 24.03 | 3.40% |
| 4 | 9.13 | 6.90% | 26 | 24.23 | 5.90% |
| 5 | 11.37 | 38.70% | 27 | 24.45 | 3.40% |
| 6 | 12.01 | 25.90% | 28 | 25.00 | 1.80% |
| 7 | 12.37 | 6.50% | 29 | 25.45 | 2.30% |
| 8 | 13.89 | 11.90% | 30 | 26.01 | 10.40% |
| 9 | 14.20 | 4.10% | 31 | 27.19 | 1.80% |
| 10 | 15.05 | 2.40% | 32 | 27.36 | 1.50% |
| 11 | 16.69 | 36.70% | 33 | 27.86 | 15.00% |
| 12 | 17.05 | 25.60% | 34 | 28.76 | 2.80% |
| 13 | 17.32 | 28.90% | 35 | 29.57 | 4.70% |
| 14 | 18.04 | 10.70% | 36 | 31.29 | 3.40% |
| 15 | 18.43 | 10.30% | 37 | 33.18 | 0.90% |
| 16 | 18.69 | 5.00% | 38 | 33.62 | 2.00% |
| 17 | 19.42 | 13.70% | 39 | 35.40 | 0.90% |
| 18 | 19.73 | 100.00% | 40 | 35.87 | 1.60% |
| 19 | 20.98 | 2.30% | 41 | 36.75 | 0.60% |
| 20 | 21.17 | 6.30% | 42 | 38.23 | 0.60% |
| 21 | 21.80 | 1.40% | 43 | 39.71 | 1.00% |
| 22 | 22.32 | 3.60% | | | |

The X-ray powder diffraction (XRPD) pattern of the crystal form I, as determined by using Cu-Kα radiation and expressed in terms of 2θ angles, is essentially as shown in FIG. 2.

The crystal form I may have a differential scanning calorimetry (DSC) curve comprising an endothermic peak at 231.1±2°C.

The differential scanning calorimetry (DSC) curve of the crystal form I is substantially as shown in FIG. 3.

The crystal form I may have a thermogravimetric analysis (TGA) curve showing no weight loss before 200±2°C.

The thermogravimetric analysis (TGA) curve of the crystal form I is substantially as shown in FIG. 3.

Provided in the present invention is a crystal form II of the nitrogen-containing spiro compound of formula A, wherein the crystal form II has an X-ray powder diffraction pattern comprising diffraction peaks at 11.32±0.20°, 11.70 ±0.20°, 11.93±0.20°, 18.03±0.20°, 18.81±0.20°, and 19.17±0.20°, as determined by using Cu-Kα radiation and expressed in terms of 2θ angles;

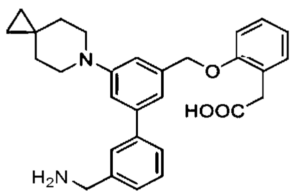

A

.

The X-ray powder diffraction (XRPD) pattern of the crystal form II, as determined by using Cu-Kα radiation and expressed in terms of 20 angles, may further comprise diffraction peaks at one or more of the following positions: 5.54 ±0.20°, 12.37±0.20°, 13.78±0.20°, 14.11±0.20°, 15.78±0.20°, and 20.80±0.20°.

The X-ray powder diffraction (XRPD) pattern of the crystal form II, as determined by using Cu-Kα radiation and expressed in terms of 20 angles, may further comprise diffraction peaks at one or more of the following positions: 13.55 ±0.20°, 16.40±0.20°, 17.74±0.20°, 20.49±0.20°, and 22.06±0.20°.

The X-ray powder diffraction (XRPD) pattern of the crystal form II, as determined by using Cu-Kα radiation and expressed in terms of 2θ angles, may comprise diffraction peaks at 5.54±0.20°, 11.32±0.20°, 11.70±0.20°, 11.93±0.20°, 12.37±0.20°, 13.55±0.20°, 13.78±0.20°, 14.11±0.20°, 15.78±0.20°, 16.40±0.20°, 17.74±0.20°, 18.03±0.20°, 18.81 ±0.20°, 19.17±0.20°, 20.49±0.20°, 20.80±0.20°, and 22.06±0.20°.

The X-ray powder diffraction (XRPD) pattern of the crystal form II, as determined by using Cu-Kα radiation and expressed in terms of 2θ angles, may comprise diffraction peaks at positions as shown in the following table:

| No. | Position in 2θ (°) | No. | Position in 2θ (°) |
|---|---|---|---|
| 1 | 5.54 | 23 | 19.17 |
| 2 | 7.20 | 24 | 19.68 |
| 3 | 8.63 | 25 | 20.21 |
| 4 | 9.04 | 26 | 20.49 |
| 5 | 11.32 | 27 | 20.80 |
| 6 | 11.70 | 28 | 21.23 |
| 7 | 11.93 | 29 | 22.06 |
| 8 | 12.37 | 30 | 22.52 |
| 9 | 12.80 | 31 | 23.15 |
| 10 | 13.55 | 32 | 23.54 |
| 11 | 13.78 | 33 | 24.44 |
| 12 | 14.11 | 34 | 24.78 |
| 13 | 14.52 | 35 | 25.33 |
| 14 | 15.23 | 36 | 25.92 |
| 15 | 15.78 | 37 | 26.40 |
| 16 | 16.40 | 38 | 26.68 |
| 17 | 16.92 | 39 | 27.52 |
| 18 | 17.31 | 40 | 28.36 |
| 19 | 17.74 | 41 | 29.62 |
| 20 | 18.03 | 42 | 30.06 |
| 21 | 18.41 | 43 | 31.61 |
| 22 | 18.81 | 44 | 34.52 |

The X-ray powder diffraction (XRPD) pattern of the crystal form II, as determined by using Cu-Kα radiation and expressed in terms of 20 angles, may comprise diffraction peaks with positions and relative intensities as shown in the following table:

| No. | Position in 20 (°) | Relative intensity | No. | Position in 20 (°) | Relative intensity |
|---|---|---|---|---|---|
| 1 | 5.54 | 62.50% | 23 | 19.17 | 100.00% |
| 2 | 7.20 | 4.60% | 24 | 19.68 | 31.90% |
| 3 | 8.63 | 19.60% | 25 | 20.21 | 11.00% |
| 4 | 9.04 | 15.80% | 26 | 20.49 | 48.80% |

(continued)

| No. | Position in 2θ (°) | Relative intensity | No. | Position in 2θ (°) | Relative intensity |
|---|---|---|---|---|---|
| 5 | 11.32 | 64.40% | 27 | 20.80 | 51.00% |
| 6 | 11.70 | 65.00% | 28 | 21.23 | 10.40% |
| 7 | 11.93 | 92.80% | 29 | 22.06 | 50.40% |
| 8 | 12.37 | 64.30% | 30 | 22.52 | 34.30% |
| 9 | 12.80 | 29.20% | 31 | 23.15 | 27.40% |
| 10 | 13.55 | 37.70% | 32 | 23.54 | 16.90% |
| 11 | 13.78 | 57.00% | 33 | 24.44 | 13.00% |
| 12 | 14.11 | 54.40% | 34 | 24.78 | 19.60% |
| 13 | 14.52 | 36.10% | 35 | 25.33 | 11.20% |
| 14 | 15.23 | 36.80% | 36 | 25.92 | 9.40% |
| 15 | 15.78 | 61.50% | 37 | 26.40 | 9.00% |
| 16 | 16.40 | 40.10% | 38 | 26.68 | 8.20% |
| 17 | 16.92 | 25.00% | 39 | 27.52 | 13.40% |
| 18 | 17.31 | 32.50% | 40 | 28.36 | 5.40% |
| 19 | 17.74 | 41.80% | 41 | 29.62 | 6.50% |
| 20 | 18.03 | 95.30% | 42 | 30.06 | 5.90% |
| 21 | 18.41 | 30.00% | 43 | 31.61 | 3.80% |
| 22 | 18.81 | 69.20% | 44 | 34.52 | 5.70% |

The X-ray powder diffraction (XRPD) pattern of the crystal form II, as determined by using Cu-Kα radiation and expressed in terms of 2θ angles, is essentially as shown in FIG. 6.

The crystal form II may have a differential scanning calorimetry (DSC) curve comprising an endothermic peak at 230.7±2°C.

The crystal form II may have a differential scanning calorimetry (DSC) curve comprising an exothermic peak at 159.1±2°C.

The differential scanning calorimetry (DSC) curve of the crystal form II is substantially as shown in FIG. 7.

The crystal form II may have a thermogravimetric analysis (TGA) curve showing a weight loss of 1.71% before 210±2°C.

The thermogravimetric analysis (TGA) curve of the crystal form II is substantially as shown in FIG. 7.

Provided in the present disclosure is a preparation method for a crystal form I of a nitrogen-containing spiro compound of formula A, comprising the following steps: dissolving the nitrogen-containing spiro compound of formula A in N-methylpyrrolidone, adding an antisolvent, and crystallizing; wherein the antisolvent is one or more selected from the group consisting of nitrile solvents, ester solvents, benzene solvents, ether solvents, and water.

The nitrile solvent may be acetonitrile.

The ester solvent may be one or more of ethyl acetate, n-propyl acetate, and isopropyl acetate, such as ethyl acetate and/or isopropyl acetate.

The benzene solvent may be one or more of toluene, ethylbenzene, xylene, isopropylbenzene, and chlorobenzene, such as toluene.

The ether solvent may be one or more of methyl tert-butyl ether, diethyl ether, cyclopentyl methyl ether, isopropyl ether, methyl tetrahydrofuran, and dioxane, such as methyl tert-butyl ether.

In the preparation method for the crystal form I, the method for the dissolving may be heating and stirring. The temperature for the heating may be 30 to 70°C, preferably 40 to 60°C, such as 50°C.

In the preparation method for the crystal form I, the volume-to-mass ratio of N-methylpyrrolidone to the nitrogen-containing spiro compound of formula A may be 15 to 50 mL/g, preferably 20 to 35 mL/g, such as 26.7 mL/g.

In the preparation method for the crystal form I, the volume-to-mass ratio of the antisolvent to the nitrogen-containing spiro compound of formula A may be 50 to 350 mL/g, preferably 80 to 250 mL/g, such as 80 mL/g, 160 mL/g, or 213.3 mL/g.

In the preparation method for the crystal form I, the volume ratio of the antisolvent to N-methylpyrrolidone may be

(1 to 10):1, preferably (3 to 8):1, such as 3:1, 6:1, or 8:1.

In the preparation method for the crystal form I, a cooling step may further be included before adding the antisolvent. The cooling step may involve cooling to between 10°C and 20°C, such as 17°C. The cooling step may have a cooling rate of 0.2°C/min.

Provided in the present disclosure is a preparation method for a crystal form II of a nitrogen-containing spiro compound of formula A, comprising the following step: stirring the nitrogen-containing spiro compound of formula A in acetonitrile for 12 to 16 days.

In the preparation method for the crystal form II, the volume-to-mass ratio of acetonitrile to the nitrogen-containing spiro compound of formula A may be 15 to 50 mL/g, preferably 25 to 40 mL/g, such as 33.3 mL/g.

In the preparation method for the crystal form II, the temperature for the stirring may be a conventional temperature for such operations in the art, preferably 15 to 30°C, such as 17°C.

In the preparation method for the crystal form II, thetime for the stirring may be 14 days.

Provided in the present disclosure is a pharmaceutical composition comprising: (1) the crystal form I or crystal form II of the nitrogen-containing spiro compound of A; and
(2) a pharmaceutically acceptable carrier.

Provided in the present disclosure is a use of the crystal form I or crystal form II of the nitrogen-containing spiro compound of formula A in the manufacture of a medicament for treating and/or preventing a complement factor D-mediated disease, wherein the complement factor D-mediated disease is a blood disease, kidney disease, cardiovascular disease, immune disorder, central nervous system disease, respiratory system disease, urogenital system disease, or ocular disease.

In the use of the crystal form I or crystal form II in the manufacture of a medicament for treating and/or preventing complement factor D-mediated diseases, the complement factor D-mediated diseases may be cold agglutinin disease, catastrophic antiphospholipid syndrome, hemolytic anemia, anti-neutrophil cytoplasmic antibody (ANCA)-associated vasculitis (AAV), warm autoimmune hemolytic anemia, paroxysmal nocturnal hemoglobinuria, IgA nephropathy, lupus nephritis, atypical hemolytic uremic syndrome, membranoproliferative glomerulonephritis (MPGN), dense deposit disease, C3 glomerulonephritis, focal segmental glomerulosclerosis, diabetic nephropathy, systemic lupus or lupus erythematosus, rheumatoid arthritis, inflammatory bowel disease, psoriasis, multiple sclerosis, organ transplant rejection, myasthenia gravis, Alzheimer's disease, respiratory distress syndrome, asthma, chronic obstructive pulmonary disease, emphysema, coronavirus infection (such as SARS-CoV, MERS-CoV, or SARS-CoV-2 infection), macular degeneration, age-related macular degeneration (AMD), macular edema, diabetic macular edema, choroidal neovascularization (CNV), uveitis, Behçet's uveitis, proliferative diabetic retinopathy, non-proliferative diabetic retinopathy, glaucoma, hypertensive retinopathy, corneal neovascularization, corneal transplant rejection, corneal dystrophy, autoimmune dry eye disease, Stevens-Johnson syndrome, Sjögren's syndrome, environmental dry eye disease, Fuchs' endothelial corneal dystrophy, retinal vein occlusion, or postoperative inflammation.

Provided in the present disclosure is a use of the crystal form I or crystal form II of the nitrogen-containing spiro compound of formula A in the manufacture of a medicament for treating and/or preventing a disease, wherein the disease is a blood disease, kidney disease, cardiovascular disease, immune disorder, central nervous system disease, respiratory disease, urogenital disease, or ocular disease.

In the use of the crystal form I or crystal form II in the manufacture of a medicament for treating and/or preventing a disease, the disease may be cold agglutinin disease, catastrophic antiphospholipid syndrome, hemolytic anemia, anti-neutrophil cytoplasmic antibody (ANCA)-associated vasculitis (AAV), warm autoimmune hemolytic anemia, paroxysmal nocturnal hemoglobinuria, IgA nephropathy, lupus nephritis, atypical hemolytic uremic syndrome, membranoproliferative glomerulonephritis (MPGN), dense deposit disease, C3 glomerulonephritis, focal segmental glomerulosclerosis, diabetic nephropathy, systemic lupus erythematosus or lupus erythematosus, rheumatoid arthritis, inflammatory bowel disease, psoriasis, multiple sclerosis, organ transplant rejection, myasthenia gravis, Alzheimer's disease, respiratory distress syndrome, asthma, chronic obstructive pulmonary disease, emphysema, coronavirus infection (such as SARS-CoV, MERS-CoV, or SARS-CoV-2 infection), macular degeneration, age-related macular degeneration (AMD), macular edema, diabetic macular edema, choroidal neovascularization (CNV), uveitis, Behçet's uveitis, proliferative diabetic retinopathy, non-proliferative diabetic retinopathy, glaucoma, hypertensive retinopathy, corneal neovascularization, corneal transplant rejection, corneal dystrophy, autoimmune dry eye disease, Stevens-Johnson syndrome, Sjögren's syndrome, environmental dry eye disease, Fuchs' endothelial corneal dystrophy, retinal vein occlusion, or postoperative inflammation.

Provided in the present disclosure is a use of the crystal form I or crystal form II of the nitrogen-containing spiro compound of formula A in the manufacture of a complement factor D inhibitor.

The complement factor D inhibitor can be used in mammalian organisms *in vivo;* it can also be employed *in vitro,* primarily for experimental purposes, such as serving as a standard or control sample for comparison, or being formulated into a kit according to conventional methods in the art to provide rapid detection of the inhibitory effects on complement factor D.

The crystal forms of the present disclosure can be identified by one or more solid-state analytical methods. For example, X-ray powder diffraction, single-crystal X-ray diffraction, differential scanning calorimetry, thermogravimetric analysis, *etc.* Those skilled in the art understand that the peak intensities and/or peak profiles in X-ray powder diffraction may vary depending on experimental conditions. Moreover, due to variations in the precision of the instruments, the measured 2$\theta$ values may have an error of approximately $\pm$0.20°. The relative intensity of peaks is more dependent on certain properties of the measured sample, such as crystal size and purity, than the peak positions. Therefore, the measured peak intensities may exhibit deviations of approximately $\pm$20%. Despite the presence of experimental errors, instrumental inaccuracies, and orientation preferences, a person skilled in the art can still obtain sufficient information from the X-ray powder diffraction data provided in the present disclosure to identify the various crystal forms. In DSC measurements, the initial temperature, peak temperature, and enthalpy of fusion of the endothermic peak obtained from actual measurements exhibit a certain degree of variability depending on the heating rate, crystal morphology, purity, and other measurement parameters.

Definition of terminology

The term "substantially" refers to the fact that the positions of the peaks in the figures may vary slightly due to minor changes in measurement equipment, measurement conditions, and variations between product batches, and should not be considered absolute values.
The term "room temperature" refers to a range of 15 to 30°C, such as 17°C or 25°C.
The term "treatment" refers to a therapeutic treatment. When referring to specific diseases or conditions, treatment means: (1) alleviating one or more biological manifestations of the disease or condition, (2) interfering with (a) one or more points in the biological cascade that causes or contributes to the condition or (b) one or more biological manifestations of the condition, (3) improving one or more symptoms, effects, or side effects associated with the condition, or one or more symptoms, effects, or side effects associated with the condition or its treatment, or (4) slowing the progression of the condition or one or more biological manifestations of the condition.
The term "prevention" refers to the reduction in the risk of acquiring or developing a disease or disorder.
The term "mammal" includes any mammal. Examples of mammals include, but are not limited to, cattle, horses, sheep, pigs, cats, dogs, mice, rats, rabbits, guinea pigs, monkeys, and humans, with humans being preferred.
Without departing from the common knowledge in the art, the above preferred conditions can be combined arbitrarily to obtain various preferred embodiments of the present disclosure.
All reagents and raw materials used in the present disclosure are commercially available.
The progressive advancement of the present disclosure lies in that the crystal forms of the nitrogen-containing spiro compound of the present disclosure have good stability, an inhibitory activity on complement factor D, a good inhibitory effect on rabbit erythrocyte hemolysis, and relatively high *in vivo* exposure and relatively high oral bioavailability.

BRIEF DESCRIPTION OF THE DRAWINGS

FIG. 1 shows an XRPD pattern of amorphous form of the compound of formula A.

FIG. 2 shows an XRPD pattern of crystal form I of the compound of formula A.

FIG. 3 shows TGA and DSC patterns of crystal form I of the compound of formula A.

FIG. 4 shows a $^1$H-NMR spectrum (DMSO-$d_6$) of crystal form I of the compound of formula A.

FIG. 5 shows a PLM pattern of crystal form I of the compound of formula A.

FIG. 6 shows an XRPD pattern of crystal form II of the compound of formula A.

FIG. 7 shows TGA and DSC patterns of crystal form II of the compound of formula A.

FIG. 8 shows a $^1$H-NMR spectrum (DMSO-$d_6$) of crystal form II of the compound of formula A.

FIG. 9 shows comparative XRPD patterns of crystal form II and crystal form I of the compound of formula A.

FIG. 10 shows comparative DSC patterns of crystal form II and crystal form I of the compound of formula A.

FIG. 11 shows comparative XRPD patterns of crystal form II after heating to 140°C and 200°C, illustrating the changes in crystal form.

FIG. 12 shows DSC and TGA patterns of the solid obtained after heating crystal form II to 140°C.

FIG. 13 shows comparative DSC patterns of the solid obtained after heating crystal form II to 140°C and 200°C.

FIG. 14 shows an XRPD pattern of the wet grinding experiment for crystal form I in ethanol.

FIG. 15 shows an XRPD pattern of the wet grinding experiment for crystal form I in water.

FIG. 16 shows an XRPD pattern of the tablet compression experiment for crystal form I.

FIG. 17 shows an HPLC chromatogram of crystal form I after being left open for 3 days.

FIG. 18 shows an XRPD pattern of crystal form I after being left open for 3 days.

FIG. 19 shows an HPLC chromatogram of crystal form I after being left open for 7 days.

FIG. 20 shows an XRPD pattern of crystal form I after being left open for 7 days.

FIG. 21 shows an HPLC chromatogram of crystal form I after being left open for 14 days.

FIG. 22 shows an XRPD pattern of crystal form I after being left open for 14 days.

FIG. 23 shows an XRPD pattern of the suspended solids of crystal form I in the biosolvent FaSSIF.

FIG. 24 shows an XRPD pattern of the suspended solids of crystal form I in the biosolvent FeSSIF.

FIG. 25 shows an XRPD pattern of the suspended solids of crystal form I in water.

FIG. 26 shows an HPLC chromatogram indicating the stability of crystal form I in the biosolvent SGF.

FIG. 27 shows an HPLC chromatogram indicating the stability of crystal form I in the biosolvent FaSSIF.

FIG. 28 shows an HPLC chromatogram indicating the stability of crystal form I in the biosolvent FeSSIF.

FIG. 29 shows an HPLC chromatogram indicating the stability of crystal form I in water.

FIG. 30 shows a DVS pattern of crystal form I.

FIG. 31 shows comparative XRPD patterns of crystal form I before and after DVS measurement.

DETAILED DESCRIPTION OF THE PREFERRED EMBODIMENT

The present disclosure is further described below by way of examples, but is not limited to the scope of the examples provided. Experimental methods without specified conditions in the following examples were carried out in accordance with conventional methods and conditions or according to the manufacturer's instructions.

**Abbreviations:**

ACN: Acetonitrile; DMSO: Dimethyl sulfoxide; IPA: Isopropyl alcohol; HPLC: High-performance liquid chromatography; NMP: N-Methylpyrrolidone; RH: Relative humidity; aw: Water activity; PLM: Polarized light microscopy; TGA: Thermogravimetric analysis; Tonset: Onset/Initial temperature. XRPD: X-ray Powder Diffraction. DSC: Differential Scanning Calorimetry; DVS: Dynamic Vapor Sorption.

**Analysis method:**

### X-ray Powder Diffraction (XRPD)

The X-ray powder diffraction pattern was obtained using a D2 Phaser from Bruker instrument with the following parameters as listed in the table below:

| Method name | 3-40+0.02+0.2+15.bsml | Induction mode | Step measurement |
|---|---|---|---|
| X-ray emitter | Cu,k-Alphal (λ=1.54184Å) | Tube voltage (kV) | 30 |
| Tube current (mA) | 10 | Divergence slit (mm) | 0.6 |
| Main optical path axial Soller slit (°) | 2.5 | Secondary optical path axial Soller slit (°) | 2.5 |
| Detector slit (°) | 5.827 | Anti-scatter slit (mm) | 0 |
| Scanning axis | θs-θd | Step angle (deg) | 0.02 |
| Dwell time per step (S) | 0.2 | Scanning range (deg) | 3-40 |

### Differential Scanning Calorimeter (DSC)

The DSC curve was obtained using a TA Instruments DSC250 model. The sample was weighed into the sample dish with precision, and the weight was recorded. Heating was performed from 25°C to 250°C at a heating rate of 10°C/min.

### Thermogravimetric Analyzer (TGA)

The TGA curve was obtained using a TA Instruments TGA550 model. The sample was weighed into the sample pan ($Al_2O_3$), and heated to 300°C at a heating rate of 10°C/min.

### $^1$H NMR

The NMR results were acquired using a Varian 400 MHz spectrometer with deuterated DMSO as the solvent.

### Polarized Light Microscope (PLM)

PLM analysis was performed using the CNOPTEC Microscope BK-Pol. A small amount of the sample was taken and placed on a glass slide. Silicone oil was added dropwise and dispersed. The sample was covered with a cover glass, and observed under a microscope.

### LCMS

The LCMS curve was acquired using a Shimadzu instrument. The methods and parameters of the instrument are shown in the table below:

LCMS method parameters

| Method name | 10-80AB_3.5min |
|---|---|
| Instrument | Shimadzu LC-20AD |
| Chromatograp hic column | Poroshell 120 SB-C18 3.0*30mm, 2.7μm |
| Column temperature | 40°C |
| Mobile phase A | 0.04% Trifluoroacetic acid in water |
| Mobile phase B | 0.02% Trifluoroacetic acid in acetonitrile |
| Flow rate | 0.8 mL/min |

(continued)

| | | | | | | |
|---|---|---|---|---|---|---|
| Gradient | Time (min) | 0.01 | 3.50 | 3.80 | 3.81 | 4.30 |
| | Mobile phase B | 10 | 80 | 80 | 10 | 10 |
| | Flow rate (mL/min) | 0.8 | 0.8 | 0.8 | 1.2 | 1.2 |
| Detection | 220 nm, 254 nm | | | | | |

**HPLC**

The analysis and detection of samples were conducted using Shimadzu instruments. Both stability and solubility experiments were measured using the following methods. The methods and parameters of the HPLC instrument are shown in the table below:

| | | | | | | | | |
|---|---|---|---|---|---|---|---|---|
| Method name | 10-80AB-35min | | | | | | | |
| Instrument | SPD-20A | | | | | | | |
| Chromatographic column | Xbridge Shield RP18 4.6*150 mm, 3.5 μm | | | | | | | |
| Column temperature | 30°C | | | | | | | |
| Mobile phase A (MPA) | 0.1% Trifluoroacetic acid in water | | | | | | | |
| Mobile phase B (MPB) | Acetonitrile | | | | | | | |
| Flow rate | 1.0 mL/min | | | | | | | |
| Diluent | Acetonitrile:water = 50:50 (V:V) | | | | | | | |
| Injection concentration | 0.25 mg/mL | | | | | | | |
| Injection volume | 10 μL | | | | | | | |
| Gradient | Time (min) | 0 | 2 | 20 | 25 | 30 | 30.5 | 35 |
| | MPA(%) | 90 | 90 | 50 | 20 | 20 | 90 | 90 |
| | MPB(%) | 10 | 10 | 50 | 80 | 80 | 10 | 10 |
| Detection | 252 nm | | | | | | | |

**Dynamic Vapor Sorption (DVS)**

The sample was analyzed using Intrinsic DVS (System Measurement System UK). The test sample, approximately 20~30 mg, was placed into the sample basket and suspended in the measurement chamber. The temperature in the test chamber was maintained at 25±1°C, while the relative humidity is increased from 0% to 90% and then decreased back to 0% at a rate of 10% per hour. Mass data was recorded every 20 seconds. Each gradient pause lasts 60 minutes.

**Example 1:** Preparation of 2-(2-((3'-(aminomethyl)-5-(6-azaspiro[2.5]octan-6-yl)-[1,1'-biphenyl]-3-yl)methoxy)phenyl) acetic acid (compound of formula A)

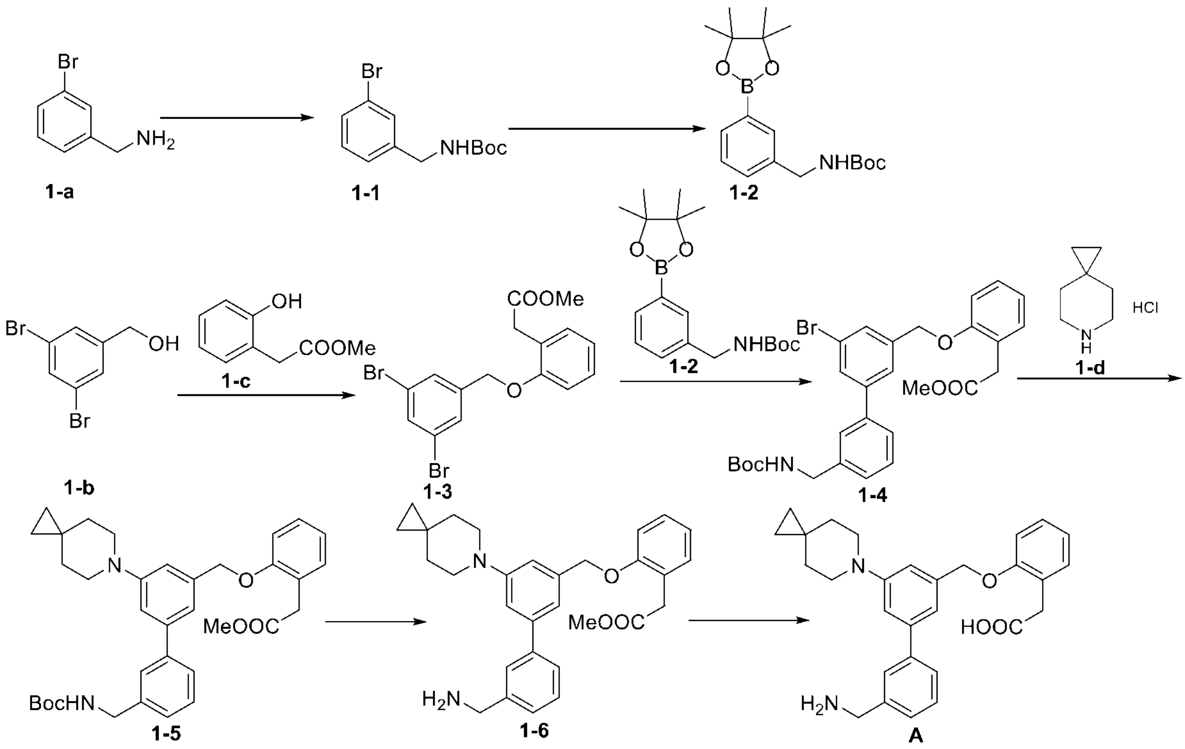


1-a 1-1 1-2 1-b 1-3 1-4 1-5 1-6 A

1.1 Synthesis of compound 1-1

Compound 1-a (11.16 g, 60 mmol), triethylamine (11.00 g, 108 mmol), and dichloromethane (120 mL) were added to a 500 mL reaction flask. Di-tert-butyl dicarbonate (15.71 g, 72 mmol) was added to the above system under an ice bath. The reaction mixture was stirred at 0°C for 1 hour, then concentrated under reduced pressure. Ethyl acetate (150 mL) and water (200 mL) were added to the residue, followed by extraction and phase separation to isolate the organic phase. The aqueous phase was extracted again with ethyl acetate (50 mL × 2). The combined organic phases were washed with saturated sodium chloride aqueous solution, dried over anhydrous sodium sulfate, and filtered. The filtrate was concentrated under reduced pressure. The residue was purified by silica gel column chromatography (petroleum ether/ethyl acetate = 10/1 (v/v)) to obtain compound 1-1.

1.2 Synthesis of compound 1-2

Compound 1-1 (18.02 g, 60 mmol), bis(pinacolato)diboron (18.28 g, 72 mmol), potassium acetate (11.78 g, 120 mmol), [1,1'-bis(diphenylphosphino)ferrocene]dichloropalladium (4.40 g, 6 mmol), and 1,4-dioxane (120 mL) were added to a 500 mL reaction flask. The reaction mixture was purged with nitrogen three times and then reacted at 80°C for 4 hours. The reaction mixture was cooled to room temperature, and filtered. The filtrate was concentrated under reduced pressure. Ethyl acetate (150 mL) and water (200 mL) were added, followed by extraction and phase separation to isolate the organic phase. The aqueous phase was extracted again with ethyl acetate (50 mL × 2). The combined organic phases were washed with saturated sodium chloride aqueous solution, dried over anhydrous sodium sulfate, and filtered. The filtrate was concentrated under reduced pressure. The residue was purified by silica gel column chromatography (petroleum ether/ethyl acetate = 8/1 (v/v)) to obtain compound 1-2. LC/MS (ESI+) m/z: $[M+H-t-Bu]^+$ = 278.15.

1.3 Synthesis of compound 1-3

Compound 1-b (5.00 g, 18.8 mmol), compound 1-c (3.12 g, 18.8 mmol), and triphenylphosphine (9.87 g, 37.6 mmol) were sequentially dissolved in dichloromethane (70 mL). A solution of bis(4-chlorobenzyl) azodicarboxylate (13.7 g, 37.6 mmol) in dichloromethane (70 mL) was added dropwise to the above system under an ice bath. After the addition was completed, the reaction was continued at 0°C for 2 hours. The reaction mixture was filtered, and the filtrate was concentrated under reduced pressure. The residue was purified by silica gel column chromatography (petroleum ether/ethyl acetate = 20/1~10/1 (v/v)) to obtain compound 1-3. LC/MS(ESI+) m/z: $[M+H]^+$=414.90.

1.4 Synthesis of compound 1-4

Compounds 1-3 (2.00 g, 4.8 mmol), 1-2 (1.30 g, 3.9 mmol), potassium carbonate (1.30 g, 9.6 mmol), and [1,1'-bis(diphenylphosphino)ferrocene]dichloropalladium (0.35 g, 0.48 mmol) were sequentially added to 1,4-dioxane/water (30 mL/3 mL). The reaction mixture was purged with nitrogen three times, and reacted at 80°C for 2 hours. After the reaction mixture was cooled to room temperature, it was filtered. The filtrate was diluted with water (30 mL) and then extracted with ethyl acetate (30 mL × 2). The combined organic phases were washed with saturated sodium chloride aqueous solution, dried over anhydrous sodium sulfate, and filtered. The filtrate was concentrated under reduced pressure. The residue was purified by silica gel column chromatography (petroleum ether/ethyl acetate = 10/1 to 5/1 (v/v)) to obtain compound 1-4. LC/MS(ESI+) m/z: $[M+H-]^+$=439.95.

1.5 Synthesis of compound 1-5

Compound 1-4 (4.3 g, 7.96 mmol), 6-azaspiro[2.5]octane hydrochloride (compound 1-d, 1.4 g, 9.55 mmol), potassium carbonate (3.3 g, 23.87 mmol), tris(dibenzylideneacetone)dipalladium (400 mg, 10 wt%), and 2-dicyclohexylphosphino-2',4',6'-triisopropylbiphenyl (400 mg, 10 wt%) were sequentially added into 1,4-dioxane (50 mL). The mixture was purged with nitrogen three times, and reacted at 90°C for 4 hours. The reaction mixture was cooled to room temperature, and filtered. The filtrate was concentrated under reduced pressure. The residue was purified by silica gel column chromatography to obtain compound 1-5. LC/MS(ESI+) m/z: $[M+H]^+$=571.15.

1.6 Synthesis of compound 1-6

Compound 1-5 (3.5 g, 6.1 mmol) was dissolved in dichloromethane (27 mL), and trifluoroacetic acid (9 mL) was added. The reaction was allowed to proceed at room temperature for 1 hour. To the reaction mixture was added a saturated aqueous solution of sodium bicarbonate to adjust the pH to 8, followed by extraction with ethyl acetate (30 mL × 3). The combined organic phases were washed with saturated brine, dried over anhydrous sodium sulfate, and filtered. The filtrate was concentrated under reduced pressure to obtain compound 1-6 (3.2 g, crude), which was used directly in the next step. LC/MS(ESI+) m/z: $[M+H]^+$=471.10.

1.7 Synthesis of compound A

Compound 1-6 (3.1 g, 6.5 mmol) and sodium hydroxide (1.05 g, 26.3 mmol) were added to a mixture of tetrahydrofuran (10 mL), methanol (5 mL), and water (5 mL). The reaction mixture was reacted at 60°C for 4 hours. The reaction solution was purified by preparative high-performance liquid chromatography, followed by lyophilization to obtain the amorphous form of compound A. Its XRPD pattern is shown in FIG. 1.

$^1$H NMR (400 MHz, DMSO-$d_6$): δ 8.27 (s, 0.59H), 8.10 (s, 1H), 7.67 (d, *J* = 7.6 Hz, 1H), 7.37-7.40 (m, 2H), 7.28 (d, *J* = 7.2 Hz, 1H), 7.08-7.14 (m, 3H), 7.00 (s , 1H), 6.92 (d, *J* = 8.0 Hz, 1H), 6.81 (t, *J* = 7.6 Hz, 1H), 5.13 (s, 2H), 3.97 (s, 2H), 3.43 (s, 2H), 3.30 (t, *J* = 5.2 Hz, 4H), 1.48 (t, *J* = 5.2 Hz, 4H), 0.35 (s, 4H); LC/MS(ESI+) m/z: $[M+H]^+$ =457.10.

**Example 2: Preparation of crystal form I of the compound of formula A**

2.1 Preparation of crystal form I

Approximately 15 mg of the amorphous form of compound A obtained in Example 1 was weighed into a sample vial. 0.5 mL of n-heptane was added, and the mixture was stirred at room temperature (approximately 17°C) and 50°C for 7 days and 14 days respectively. The resulting solids were then subjected to XRPD analysis. The results indicated that all the obtained solids were of crystal form I. The specific experimental conditions and results are provided in Tables 1, 3, and 4.

Table 1. Conditions and results for crystallization experiment using amorphous form of compound of formula A as starting material in n-heptane

| Temperature | Solvent | Result (after stirring for 7 days) | Result (after stirring for 14 days) |
|---|---|---|---|
| Room temperature | n-Heptane | Crystal form I | Crystal form I |
| 50°C | | | |

2.2 Preparation of crystal form I

Approximately 15 mg of the amorphous form of compound A obtained in Example 1 was weighed into a sample vial. At 50°C, 0.4 mL of N-methylpyrrolidone was added to completely dissolve the amorphous form of compound A. The mixture was brought to room temperature (approximately 17°C), and a certain volume of antisolvent was added, followed by stirring until solids precipitated. The results indicated that all the obtained solids were of crystal form I. The specific experimental conditions and results are provided in Tables 2, 3, and 4.

Table 2. Conditions and results for antisolvent crystallization experiment using amorphous form of compound of formula A as starting material

| No. # | Anti-solvent | Total volume (Good solvent: Anti-solvent) | Result |
|---|---|---|---|
| 1 | Acetonitrile, 2.4 mL | 2.8 mL (1:6) | Crystal form I |
| 2 | Toluene, 3.2 mL | 3.6 mL (1:8) | |
| 3 | Ethyl acetate, 3.2 mL | 3.6 mL (1:8) | |
| 4 | Isopropyl acetate, 3.2 mL | 3.6 mL (1:8) | |
| 5 | Methyl tert-butyl ether, 3.2 mL | 3.6 mL (1:8) | |
| 6 | Water, 1.2 mL | 1.6 mL (1:3) | |

2.3 The characterization data of the crystal form I are shown in Table 3 below, and the XRPD pattern analysis data of the crystal form I are shown in Table 4.

Table 3. Characterization data of crystal form I

| **Parameter** | **Method** | **Result** |
|---|---|---|
| X-ray Powder Diffraction (XRPD) | 3-40° (2θ) | High crystallinity, as shown in FIG. 2; |
| Thermogravimetric Analysis | TGA, 10°C/min | No weight loss at 200°C, as shown in FIG. 3; |
| Thermal phenomena | DSC, 10°C/min | The melting point was 231.06°C, and the peak temperature was 231.05°C, as shown in FIG. 3; |
| Residual solvent | $^1$H-NMR (DMSO-$d_6$) | No residual solvent, as shown in FIG. 4; |
| Morphology | PLM | Irregularly shaped crystals, as shown in FIG. 5; |

Table 4. XRPD pattern analysis data of crystal form I

| **No.** | **2θ (°)** | **d-value (Å)** | **Relative intensity** | **No.** | **2θ (°)** | **d-value (Å)** | **Relative intensity** |
|---|---|---|---|---|---|---|---|
| 1 | 3.498 | 25.24141 | 3.00% | 23 | 22.935 | 3.87446 | 10.60% |
| 2 | 5.665 | 15.58919 | 45.80% | 24 | 23.412 | 3.79667 | 25.50% |
| 3 | 6.879 | 12.83883 | 3.00% | 25 | 24.027 | 3.70085 | 3.40% |
| 4 | 9.126 | 9.68309 | 6.90% | 26 | 24.231 | 3.67012 | 5.90% |
| 5 | 11.365 | 7.77975 | 38.70% | 27 | 24.447 | 3.63822 | 3.40% |
| 6 | 12.01 | 7.36292 | 25.90% | 28 | 25 | 3.5589 | 1.80% |
| 7 | 12.372 | 7.1488 | 6.50% | 29 | 25.453 | 3.49661 | 2.30% |
| 8 | 13.892 | 6.36972 | 11.90% | 30 | 26.005 | 3.4236 | 10.40% |
| 9 | 14.203 | 6.23094 | 4.10% | 31 | 27.194 | 3.27665 | 1.80% |
| 10 | 15.048 | 5.88288 | 2.40% | 32 | 27.356 | 3.25755 | 1.50% |
| 11 | 16.693 | 5.30646 | 36.70% | 33 | 27.856 | 3.20021 | 15.00% |
| 12 | 17.045 | 5.19785 | 25.60% | 34 | 28.761 | 3.10155 | 2.80% |
| 13 | 17.317 | 5.1169 | 28.90% | 35 | 29.571 | 3.01838 | 4.70% |

(continued)

| No. | 2θ (°) | d-value (Å) | Relative intensity | No. | 2θ (°) | d-value (Å) | Relative intensity |
|---|---|---|---|---|---|---|---|
| 14 | 18.038 | 4.91391 | 10.70% | 36 | 31.294 | 2.85603 | 3.40% |
| 15 | 18.434 | 4.80926 | 10.30% | 37 | 33.181 | 2.69779 | 0.90% |
| 16 | 18.692 | 4.74339 | 5.00% | 38 | 33.623 | 2.66335 | 2.00% |
| 17 | 19.423 | 4.56651 | 13.70% | 39 | 35.403 | 2.53342 | 0.90% |
| 18 | 19.728 | 4.49657 | 100.00% | 40 | 35.873 | 2.5013 | 1.60% |
| 19 | 20.983 | 4.2303 | 2.30% | 41 | 36.754 | 2.44333 | 0.60% |
| 20 | 21.169 | 4.19355 | 6.30% | 42 | 38.227 | 2.35251 | 0.60% |
| 21 | 21.801 | 4.07351 | 1.40% | 43 | 39.707 | 2.26813 | 1.00% |
| 22 | 22.322 | 3.97957 | 3.60% | | | | |

**Example 3: Preparation of crystal form II of the compound of formula A**

3.1 Preparation of crystal form II

Approximately 15 mg of the amorphous form of compound A obtained in Example 1 was weighed into a sample vial, and 0.5 mL of acetonitrile was added. The mixture was stirred at room temperature (approximately 17°C) for 14 days, after which the resulting solid product was subjected to XRPD analysis. The results indicated that the obtained solids were of crystal form II.

3.2 The characterization data of the crystal form II are shown in Table 5 below, and the XRPD pattern analysis data of the crystal form II are shown in Table 6.

Table 5. Characterization data of crystal form II

| Parameter | Method | Result |
|---|---|---|
| X-ray Powder Diffraction (XRPD) | 3-40° (2θ) | High crystallinity, as shown in FIG. 6; |
| Thermogravimetric Analysis | TGA, 10°C/min | A weight loss of 1.71% prior to 210°C, as shown in FIG. 7. |
| Thermal phenomena | DSC, 10°C/min | An endothermic peak observed at 230.69°C, and an exothermic peak observed at 159.11°C, as shown in FIG. 7. |
| Residual solvent | $^{1}$H-NMR (DMSO-$d_6$) | As shown in FIG. 8. |

Table 6. XRPD pattern analysis data of crystal form II

| No. | 2θ (°) | d-value (Å) | Relative intensity | No. | 2θ (°) | d-value (Å) | Relative intensity |
|---|---|---|---|---|---|---|---|
| 1 | 5.539 | 15.9435 | 62.50% | 23 | 19.171 | 4.62587 | 100.00% |
| 2 | 7.195 | 12.27668 | 4.60% | 24 | 19.676 | 4.50837 | 31.90% |
| 3 | 8.63 | 10.23797 | 19.60% | 25 | 20.214 | 4.3896 | 11.00% |
| 4 | 9.042 | 9.77248 | 15.80% | 26 | 20.491 | 4.33081 | 48.80% |
| 5 | 11.322 | 7.80895 | 64.40% | 27 | 20.795 | 4.26813 | 51.00% |
| 6 | 11.695 | 7.56096 | 65.00% | 28 | 21.228 | 4.18215 | 10.40% |
| 7 | 11.934 | 7.40997 | 92.80% | 29 | 22.057 | 4.02677 | 50.40% |
| 8 | 12.373 | 7.14799 | 64.30% | 30 | 22.521 | 3.94473 | 34.30% |
| 9 | 12.797 | 6.91228 | 29.20% | 31 | 23.153 | 3.83851 | 27.40% |
| 10 | 13.549 | 6.53019 | 37.70% | 32 | 23.535 | 3.77713 | 16.90% |
| 11 | 13.775 | 6.42328 | 57.00% | 33 | 24.435 | 3.63997 | 13.00% |

(continued)

| No. | 2θ (°) | d-value (Å) | Relative intensity | No. | 2θ (°) | d-value (Å) | Relative intensity |
|---|---|---|---|---|---|---|---|
| 12 | 14.108 | 6.27274 | 54.40% | 34 | 24.778 | 3.59035 | 19.60% |
| 13 | 14.516 | 6.09714 | 36.10% | 35 | 25.329 | 3.51351 | 11.20% |
| 14 | 15.229 | 5.81319 | 36.80% | 36 | 25.918 | 3.43499 | 9.40% |
| 15 | 15.776 | 5.61279 | 61.50% | 37 | 26.403 | 3.37295 | 9.00% |
| 16 | 16.401 | 5.40047 | 40.10% | 38 | 26.682 | 3.33835 | 8.20% |
| 17 | 16.919 | 5.23617 | 25.00% | 39 | 27.516 | 3.23897 | 13.40% |
| 18 | 17.314 | 5.11775 | 32.50% | 40 | 28.357 | 3.14484 | 5.40% |
| 19 | 17.742 | 4.9951 | 41.80% | 41 | 29.617 | 3.01384 | 6.50% |
| 20 | 18.031 | 4.91578 | 95.30% | 42 | 30.056 | 2.97077 | 5.90% |
| 21 | 18.407 | 4.81616 | 30.00% | 43 | 31.614 | 2.82785 | 3.80% |
| 22 | 18.811 | 4.7136 | 69.20% | 44 | 34.522 | 2.59599 | 5.70% |

**Example 4: Comparison and study on transformation relationship between crystal form I and crystal form II**

4.1 Comparison of spectral data between crystal form I and crystal form II

When comparing the crystal form II with the crystal form I, distinct differences are observed in the 2θ angle ranges of 16-18° and 22-24° in their spectra, as shown in the boxed 2θ angle regions of the comparative XRPD patterns of the crystal form II and the crystal form I in FIG. 9. The endothermic peak temperatures of both forms are similar, but the crystal form II exhibits an exothermic peak at 159°C, as shown in the comparative DSC patterns in FIG. 10.

4.2 Heating experiment of crystal form II

A heating experiment was conducted on the crystal form II. The crystal form II sample was heated to 140°C using TGA assay and to 200°C using DSC assay. The resulting solids were subsequently characterized by XRPD, TGA, DSC, and $^1$H NMR testings. The test results showed that when heated to 140°C, the XRPD pattern indicated no change in the crystal form, no weight loss was observed in the TGA, no solvent residue peaks of acetonitrile were detected in the $^1$H NMR spectrum, and an exothermic peak at 159 °C was still observable in the DSC pattern. When heated to 200°C, the XRPD pattern indicated that the obtained solid was crystal form I. No solvent residue peaks of acetonitrile were observed in the $^1$H NMR spectrum, and the endothermic peak at 159°C in the DSC pattern disappeared. Therefore, it can be concluded that the exothermic peak at 159°C corresponds to the crystal transformation peak, while the endothermic peak at 230°C corresponds to the melting peak of crystal form I. The comparative XRPD patterns for the heating experiment are shown in FIG. 11. The DSC and TGA patterns of the solid obtained after heating to 140°C are shown in FIG. 12. The comparative DSC patterns for the heating experiment are shown in FIG. 13.

**Effect example 1: Evaluation of crystal forms**

**1. Water activity experiment**

Organic solvent and water were mixed at molar ratios of 0.001:0.999, 0.2:0.8, 0.5:0.5, 0.8:0.2, and 0.999:0.001. The resulting crystal form I from Example 2 was added to the mixture to achieve a suspension state. The mixtures were stirred at 25°C and 50°C for 30 minutes, respectively, followed by filtration, and the filtrate was collected. Approximately 10 mg of the crystal form I obtained in Example 2 was weighed and added to the filtrate. The results were analyzed after stirring for 5 days and 11 days, respectively. The results indicate that the solids obtained under the following conditions were all of crystal form I. The specific experimental conditions and results are provided in Tables 7 and 8.

Table 7. Experimental conditions and results of water activity at 25°C

| No. # | Solvent | Molar ratio (Organic solvent: water) | aw | Result (5 days) | Result (11 days) |
|---|---|---|---|---|---|
| 1 | ACN-$H_2O$ | 0.001:0.999 | 1.00 | Crystal form I | Crystal form I |
| 2 | | 0.2:0.8 | 0.91 | | |
| 3 | | 0.5:0.5 | 0.90 | | |
| 4 | DMSO-$H_2O$ | 0.001:0.999 | 0.999 | Crystal form I | Crystal form I |
| 5 | | 0.2:0.8 | 0.70 | | |
| 6 | | 0.5:0.5 | 0.28 | | |
| 7 | | 0.8:0.2 | 0.07 | | |
| 8 | | 0.999:0.001 | 0.000 3 | | |
| 9 | IPA-$H_2O$ | 0.001:0.999 | 0.99 | Crystal form I | Crystal form I |
| 10 | | 0.2:0.8 | 0.94 | | |
| 11 | | 0.5:0.5 | 0.89 | | |
| 12 | | 0.8:0.2 | 0.52 | | |
| 13 | | 0.999:0.001 | 0.003 | | |

Table 8. Experimental conditions and results of water activity at 50°C

| No. # | Solvent | Molar ratio (Organic solvent: water) | aw | Result (5 days) | Result (11 days) |
|---|---|---|---|---|---|
| 1 | ACN-$H_2O$ | 0.001:0.999 | 1.00 | Crystal form I | Crystal form I |
| 2 | | 0.2:0.8 | 0.91 | | |
| 3 | | 0.5:0.5 | 0.90 | | |
| 4 | | 0.8:0.2 | 0.78 | | |
| 5 | | 0.999:0.001 | 0.01 | | |
| 6 | DMSO-$H_2O$ | 0.001:0.999 | 0.999 | Crystal form I | Crystal form I |
| 7 | | 0.2:0.8 | 0.70 | | |
| 8 | | 0.5:0.5 | 0.28 | | |
| 9 | | 0.8:0.2 | 0.07 | | |
| 10 | | 0.999:0.001 | 0.000 3 | | |
| 11 | IPA-$H_2O$ | 0.001:0.999 | 0.99 | Crystal form I | Crystal form I |
| 12 | | 0.2:0.8 | 0.94 | | |
| 13 | | 0.5:0.5 | 0.89 | | |
| 14 | | 0.8:0.2 | 0.52 | | |
| 15 | | 0.999:0.001 | 0.003 | | |

**2. Grinding and crystal transformation experiment**

2.1 Wet grinding

Approximately 20 mg of the crystal form I obtained in Example 2 was weighed into a mortar. 0.04 mL of water or ethanol was added, and manual grinding was performed for 2 min, 5 min, and 10 min, respectively. After each grinding period, XRPD was measured. If the solvent completely evaporates during the grinding process, an additional 0.04 mL of water or ethanol should be added to the mortar each time. The results indicated that the crystal form I remained unchanged after grinding for 10 minutes, with no significant decrease in crystallinity observed. The XRPD pattern of the crystal form I subjected to wet grinding in ethanol is shown in FIG. 14, and the XRPD pattern of the crystal form I subjected to wet grinding in water is shown in FIG. 15.

2.2 Tablet compression experiment

Approximately 30 mg of the crystal form I obtained in Example 2 was weighed, compressed into a tablet at 40 MPa, and subjected to XRPD testing. The results indicated that the crystal form I remained unchanged after compression, with no significant decrease in crystallinity observed. The XRPD results are shown in FIG. 16.

**3. Stability experiment**

An appropriate amount of the crystal form I obtained in Example 2 was weighed into a sample vial and placed under open conditions at 60°C, 25°C/60% RH, 40°C/75% RH, and 25°C/90(±5)% RH for 3 days, 7 days, and 14 days, respectively. The purity was determined by HPLC. The results indicated that after being stored under the above conditions for 14 days, the main peak purity did not show significant changes, and the crystal form remained unchanged. The stability conditions and results are shown in Table 9 below. The HPLC chromatogram of the crystal form I after being exposed for 3 days is shown in FIG. 17, and the XRPD pattern of the crystal form I after being exposed for 3 days is shown in FIG. 18. The HPLC chromatogram of the crystal form I after being exposed for 7 days is shown in FIG. 19, and the XRPD pattern of the crystal form I after being exposed for 7 days is shown in FIG. 20. The HPLC chromatogram of the crystal form I after being exposed for 14 days is shown in FIG. 21, and the XRPD pattern of the crystal form I after being exposed for 14 days is shown in FIG. 22.

Table 9. Stability experiment results

| Conditions | 0 days | 3 days | 7 days | 14 days |
|---|---|---|---|---|
| 60°C | 99.33% | 99.36% | 98.92% | 99.44% |
| 25°C/60% RH | | 99.34% | 98.95% | 99.43% |
| 40°C/75% RH | | 99.30% | 99.00% | 99.48% |
| 25°C/90%(±5%) RH | | 99.18% | 98.96% | 99.52% |

Experimental conclusion: After storage under the above conditions for 14 days, the main peak purity of the crystal form I did not show significant changes, and the crystal form remained unaltered, indicating good stability.

**4. Experiments on solubility and stability in biosolvents and water**

4.1 Solubility experiments in biosolvents and water

Approximately 5 mg of the crystal form I obtained in Example 2 was weighed into a sample vial, and SGF, FaSSIF, FeSSIF, and water were added separately to prepare suspensions with a concentration of 5 mg/mL. The samples were fully stirred in SGF, FaSSIF, FeSSIF, and water for 0.5 hours, 2 hours, and 24 hours before being withdrawn. Their solubility was determined using HPLC, and the solid form in the suspensions was analyzed by XRPD for its crystal form. The crystal form I was completely dissolved in SGF and remained in suspension in FaSSIF, FeSSIF, and water. The crystal form I exhibits relatively high solubility in SGF (>5 mg/mL), while its solubility in FaSSIF, FeSSIF, and water is less than 0.73 mg/mL. The crystal form remained unchanged after stirring for 24 hours in FaSSIF, FeSSIF, and water. The solubility results are shown in Table 10. The XRPD pattern for the suspended solid of the crystal form I in the biosolvent FaSSIF is shown in FIG. 23. The XRPD pattern for the suspended solid of the crystal form I in the biosolvent FeSSIF is shown in FIG. 24. The XRPD pattern for the suspended solid of the crystal form I in water is shown in FIG. 25.

Table 10. Results for solubility experiments in biosolvents and water

| Crystal form I solubility (mg/mL) | | | |
|---|---|---|---|
| Conditions | 0.5 h | 2h | 24 h |
| SGF | >5 | >5 | >5 |
| FaSSIF | 0.054 | 0.066 | 0.075 |
| FeSSIF | 0.63 | 0.70 | 0.73 |

(continued)

| Crystal form I solubility (mg/mL) | | | |
|---|---|---|---|
| Conditions | 0.5 h | 2h | 24 h |
| Water | LOQ | 0.00020 | 0.00058 |
| Note: LOQ stands for limit of quantitation, which refers to the lowest amount of the analyte in a sample that can be quantitatively determined. | | | |

4.2 Determination of stability in biosolvents

Approximately 2.5 mg of the crystal form I obtained in Example 2 was weighed and added to 0.5 mL of SGF, FaSSIF, FeSSIF, and water, respectively. After thorough stirring for 0.5 hours, 2 hours, and 24 hours, the mixed solutions were diluted to 10 mL, and their purity was determined using HPLC. The results indicated that the crystal form I did not exhibit a significant reduction in main peak purity in SGF, FaSSIF, or water. However, in FeSSIF, the main peak purity gradually decreased with increasing stirring time, declining from 99.56% to 98.04%. The stability conditions and results are shown in Table 11. The HPLC chromatogram for the stability of the crystal form I in the biosolvent SGF is shown in FIG. 26. The HPLC chromatogram for the stability of the crystal form I in the biosolvent FaSSIF is shown in FIG. 27. The HPLC chromatogram for the stability of the crystal form I in the biosolvent FeSSIF is shown in FIG. 28. The HPLC chromatogram for the stability of the crystal form I in water is shown in FIG. 29. Main impurities are indicated by the boxes in FIGs. 26 to 29.

Table 11. Determination results of stability in biosolvents

| HPLC main peak purity (%) | | | | |
|---|---|---|---|---|
| Conditions | 0 h | 0.5 h | 2 h | 24 h |
| SGF | 99.33 | 99.13 | 99.10 | 99.08 |
| FaSSIF | | 98.97 | 98.91 | 98.86 |
| FeSSIF | | 99.50 | 99.34 | 98.04 |
| Water | | 99.06 | 98.79 | 98.77 |

**5. Hygroscopicity experiment**

DVS was used to determine the hygroscopicity of the crystal form I obtained in Example 2, and XRPD analysis was performed on the solid before and after the DVS measurement. The results indicated that crystal form I exhibited slight hygroscopicity, with a weight gain of 0.55% under 0-80% RH conditions. No change in crystal form was observed before and after the DVS measurement. The DVS results are shown in FIG. 30, and the comparative XRPD patterns of the crystal form I before and after DVS measurement are shown in FIG. 31.

**6. Stability experiment of crystal form I in DMSO under high-temperature conditions**

Approximately 100 mg of the crystal form I obtained in Example 2 was weighed into a sample vial and suspended by the addition of DMSO (2 mL). The suspension was stirred at 50°C for 24 hours and 48 hours, respectively. The solid and filtrate were then analyzed by LCMS. The results indicated that after stirring in DMSO for 48 hours, the crystal form I exhibited no significant change in solid purity, and a small amount of impurities appeared in the filtrate. The measured purities are shown in Table 12 below.

Table 12. Results for stability experiment of crystal form I in DMSO under high-temperature conditions

| No. # | Sample name | Purity by LCMS (254 nm) |
|---|---|---|
| 1 | Crystal form I | 99.06% |
| 2 | Stirred in DMSO system for 24 hours (solid) | 99.48% |
| 3 | Stirred in DMSO system for 24 hours (filtrate) | 98.71 % |
| 4 | Stirred in DMSO system for 48 hours (solid) | 98.92% |

(continued)

| No. # | Sample name | Purity by LCMS (254 nm) |
|---|---|---|
| 5 | Stirred in DMSO system for 48 hours (filtrate) | 97.14% |

Experimental conclusion: The purity of the crystal form I of the present disclosure exhibited relatively small changes under high-temperature conditions, demonstrating good stability.

**Effect example 2: Biological activity assay**

**1. *In vitro* screening assay for complement factor D inhibitory activity (C3b method)**

1.1 Experimental materials and instruments

V-bottom plates (AXYGEN), DMSO (Sigma), MicroVue Bb Plus ELISA kit (Quidel), Complement Factor C3b (abbreviated as Factor C3b, Complement tech), Complement Factor B (abbreviated as Factor B, Complement tech), Complement Factor D (abbreviated as Factor D, Complement tech), EDTA (Macklin), GVB° (Complement tech), EGTA (Aladdin), $MgCl_2$ (Aladdin), NaOH (Sinopharm), microplate reader (Molecular Devices, SpectraMax i3x), microplate incubator shaker (Thermo, MB100-2A), pipettes (Gilson).

1.2 Pre-experiment preparation

1.2.1 Preparation of Mg-EGTA

0.1 M Mg-EGTA: 3.8 g of EGTA, 0.9521 g of $MgCl_2$, and 0.7 g of NaOH were weighed. The pH was adjusted to 7.5 using NaOH, and the volume was brought to 100 mL with distilled water. The solution was filtered through a 0.2 μm sterile filter, aliquoted, and stored at 4°C.

1.2.2 Preparation of working solution

Buffer: 0.1 M Mg-EGTA solution was diluted 10-fold with GVB° buffer to 10 mM.
Factor B working solution: A 1 mg/mL Factor B solution (10.75 μM) was diluted 6.7-fold with buffer to a concentration of 1.6 μM.
C3b working solution: A 1 mg/mL C3b solution (5.68 μM) was diluted 5-fold with buffer to a concentration of 1.12 μM.
Factor D working solution: A 0.1 mg/mL Factor D solution (4.17 μM) was diluted 1303-fold with buffer to a concentration of 3.2 nM.
Note: a. The above dilution factors are for reference only; adjust according to the actual concentration indicated on the reagent.

b. Factor B: 93 kDa, Factor D: 24 kDa, Factor C3b: 176 kDa.

1.2.3 Preparation of stop solution

Stop solution: An appropriate amount of EDTA powder was weighed and dissolved in a certain volume of GVB° buffer. The pH was adjusted to 7.5 using NaOH, and the mixture was stirred until clear to prepare a 10 mM solution.

1.2.4 Compound preparation

Compound stock solution: A 40 mM compound solution was diluted with DMSO to prepare a 1 mM stock solution. The 1 mM stock solution was then diluted 3-fold with DMSO across 8 concentration points to generate compound stock solutions at various concentrations.
Compound working solution: The stock solution was diluted 250-fold with buffer to obtain solutions of each compound concentration.

1.3 Experimental procedure

In the V-bottom plate, for the experimental group 10 μL of Factor D solution and 10 μL of compound solution were added; for the positive control group 10 μL of Factor D solution and buffer containing 0.4% DMSO were added; for the blank control group 20 μL of buffer containing 0.2% DMSO was added. The mixture was incubated at 37°C for 15 minutes. Factor B working solution and Factor C3b working solution were mixed at a 1:1 ratio, and 20 μL of the mixture was added to each well, followed by incubation at 37°C for 30 minutes. The reaction was terminated by adding 40 μL of stop solution. The production of Bb was detected using the MicroVue Bb Plus ELISA kit.

1.4 ELISA testing

1.4.1 The required microplate wells were removed and allowed to equilibrate to room temperature, while the remaining wells were repackaged and stored at 4°C.
1.4.2 The wells were washed twice with 300 μL of 1× Wash Buffer, and incubated at 25°C for 1 minute during the first wash.
1.4.3 The samples were diluted 8-fold with Complement Specimen Diluent, and 100 μL of each sample was added per well, followed by incubation at 25°C for 30 minutes.
1.4.4 The liquid in the wells was discarded, and the wells were washed 5 times with 300 μL of 1× Wash Buffer. The wells were incubated at room temperature for 1 minute during the first wash.
1.4.5 50 μL of Bb Plus Conjugate was added to each well and incubated at 25°C for 30 minutes.
1.4.6 The liquid in the wells was discarded, and the wells were washed 5 times with 300 μL of 1× Wash Buffer. The wells were incubated at room temperature for 1 minute during the first wash.
1.4.7 100 μL of compound working solution (or substrate solution) was added to each well and incubated at 25°C for 15 minutes.
1.4.8 100 μL of stop solution was added to each well, and the absorbance at 450 nm was measured within 30 minutes.

1.5 Data analysis

1.5.1 Inhibition rates at various drug concentrations:

$$\text{Inhibition rate}(\%) = \frac{\text{PC group OD value} - \text{treatment group OD value}}{\text{PC group OD value} - \text{NC group OD value}} \times 100\%$$

PC group represents 0% inhibition rate, and NC group represents 100% inhibition rate.

1.5.2 Calculation of Signal-to-Background ratio (S/B):

The ratio of the OD average of the PC group to that of the NC group represents the size of the signal window.

1.5.3 Z'-factor:

Calculation formula:

$$Z' \text{ factor} = 1 - \frac{3 \times \text{PC group OD SD} + 3 \times \text{NC group OD SD}}{|\text{PC group OD mean} - \text{NC group OD mean}|}$$

The Z'-factor should be greater than 0.4.

1.5.4 $IC_{50}$ of the compounds

$IC_{50}$: half maximal inhibitory concentration, representing the concentration at which a compound inhibits 50% of

the enzymatic activity of complement factor D.

The data were collected to calculate the inhibition rate and the logarithm of the compound concentration, and the $IC_{50}$ value was calculated using GraphPad Prism software. The inhibitory activity of the compound of formula A obtained in Example 1 of the present disclosure on complement factor D is shown in Table 13 below:

Table 13. Inhibitory activity of compound of formula A on complement factor D

| Compound | C3b $IC_{50}$ (nM) |
|---|---|
| Compound of formula A | 10 |

Experimental conclusion: The compound of formula A of the present disclosure exhibited excellent inhibitory activity on complement factor D.

**2. Evaluation of compound inhibition activity on the alternative pathway by rabbit erythrocyte hemolysis assay**

2.1 Experimental materials and instruments

Normal Human Serum (NHS) (collected from healthy individuals), Normal Human Plasma (NHP) (Shanghai Yuduo), 96-well enzyme-linked immunosorbent assay (ELISA) plates (Jet Biofil), Japanese big-ear white rabbits (Wuhan WQJX Biotechnology), Alsever's solution (Procell), centrifuge (Thermo, PICO17), constant temperature shaker (Shanghai Fuma), decolorization shaker (Beijing Liuyi Biotechnology), cell counter (Invitrogen, Counter Countess II). GVB°, EGTA, $MgCl_2$, NaOH, microplate reader, microplate thermostatic shaker, pipettes, etc., were the same as Experiment 1.

2.2 Preparation of working solution

48% NHP: 100% NHP was diluted to a concentration of 48% with buffer.

26.4% NPS: 100% NHS was diluted to a concentration of 26.4% with buffer.

Rabbit erythrocyte suspension: Blood was collected from the marginal ear vein of rabbits, anticoagulated with Alsever's solution at a 1:1 ratio, aliquoted, and stored at 4°C for up to 4 weeks. Prior to use, the sample was centrifuged at 500g for 5 minutes, and the Alsever's solution was discarded. It was then washed three times with an equal volume of buffer by centrifugation at 500g for 5 minutes each time. Finally, its density was adjusted to $6\times10^8$ cells/mL using buffer.

Compound stock solution: A 40 mM compound solution was diluted with DMSO to prepare a 2 mM stock solution. The 2 mM stock solution was then diluted 3-fold with DMSO across 10 concentration points to generate compound stock solutions at various concentrations.

Compound working solution: The stock solution was diluted 500-fold with buffer to obtain solutions of each compound concentration.

2.3 Experimental procedure

In a 96-well microplate, for the experimental group 50 μL of 48% NHP or 26.4% NHS and 50 μL of compound solution were added; for the positive control group 50 μL of 48% NHP or 26.4% NHS and 50 μL of buffer containing 0.2% DMSO were added; for the blank control group 50 μL of 48% inactivated NHP or 26.4% inactivated NHS and 50 μL of buffer containing 0.2% DMSO were added; for the $H_2O$ group 100 μL of double-distilled water was added. The plate was incubated at 37°C for 15 minutes. Then, 20 μL of rabbit erythrocyte suspension was added to each well, followed by incubation at 37°C on a shaker for 30 minutes. The plate was centrifuged at 2000g (3380 rpm) for 5 minutes, and 100 μL of supernatant was transferred to a new 96-well microplate, after which absorbance was measured at 415 nm.

2.4 Data analysis

2.4.1 Hemolysis percentage at each drug concentration:

$$\text{Hemolysis percentage}(\%) = \frac{\text{treatment group OD value} - \text{NC group OD value}}{\text{PC group OD value} - \text{NC group OD value}} \times 100\%$$

The PC group represents 100% hemolysis, and the NC group represents 0% hemolysis.

2.4.2 Calculation of Signal-to-Background ratio (S/B):

The ratio of the OD average of the PC group to that of the NC group represents the size of the signal window.

2.4.3 Z'-factor:

Calculation formula:

$$Z' \text{ factor} = 1 - \frac{3 \times \text{PC group OD SD} + 3 \times \text{NC group OD SD}}{|\text{PC group OD mean} - \text{NC group OD mean}|}$$

2.4.4 $IC_{50}$ of the compounds

$IC_{50}$: Half maximal inhibitory concentration. The data were collected to calculate the hemolysis percentage and the logarithm of the compound concentration, and the $IC_{50}$ value was calculated using GraphPad Prism software. The inhibitory activity of the compound of formula A obtained in Example 1 of the present disclosure on rabbit erythrocyte hemolysis is shown in Table 14 below:

Table 14. Inhibitory activity of compound of formula A on rabbit erythrocyte hemolysis

| Compound | $IC_{50}$ (nM) of rabbit erythrocyte hemolysis |
|---|---|
| Compound of formula A | 41 |

Experimental conclusion: The compound of formula A of the present disclosure exhibited excellent inhibitory effects on rabbit erythrocyte hemolysis.

**3. *In vivo* pharmacokinetic study of the compound of formula A**

After adaptive acclimation, SPF-grade SD rats were administered 1 mg/kg of the compound of formula A obtained in Example 1 of the present disclosure via either single oral gavage or tail vein injection. Plasma was collected at specific time points post-dose, and the concentration of the compound in plasma was determined by LC-MS/MS (AB SCIEX Qtrap4500). PK parameters of the compound were calculated using software to reflect its *in vivo* pharmacokinetic properties in animals. PK parameters for the compound of formula A of the present disclosure are shown in Table 15 below:

Table 15. PK parameters of compound of formula A

| Compound | Dosage of administration mg/kg | Mode of administration | $C_{max}$ (ng/mL) | $T_{1/2}$ (hr) | $AUC_{last}$ (ng/mL*hr) | Oral bioavailability F (%) |
|---|---|---|---|---|---|---|
| Compound of formula A | 1 | PO (3% DMSO + 4% Tween) | 577±12.3 | 4.13±1.38 | 3238±115 | 40.3% |
| | | IV | 1803±227 | 3.59±0.18 | 8031±574 | / |

Experimental conclusion: The compound of formula A of the present disclosure achieved relatively high *in vivo* exposure and relatively high oral bioavailability at relatively low doses, demonstrating overall favorable pharmacokinetic properties.

## Claims

**1.** A crystal form I of a nitrogen-containing spiro compound of formula A, wherein the crystal form I has an X-ray powder

diffraction pattern comprising diffraction peaks at 5.67±0.20°, 11.37±0.20°, 16.69±0.20°, 17.32±0.20°, and 19.73 ±0.20°, as determined by using Cu-Kα radiation and expressed in terms of 2θ angles;

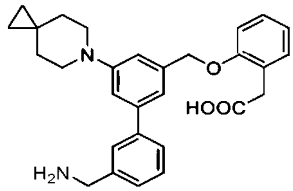

A

2. The crystal form I according to claim 1, wherein the crystal form I satisfies one or more of the following conditions:

(1) the X-ray powder diffraction pattern of the crystal form I, as determined by using Cu-Kα radiation and expressed in terms of 2θ angles, further comprises diffraction peaks at one or more of the following positions: 12.01±0.20°, 13.89±0.20°, 17.05±0.20°, 19.42±0.20°, 23.41±0.20°, and 27.86±0.20°;

the X-ray powder diffraction pattern of the crystal form I, as determined by using Cu-Kα radiation and expressed in terms of 20 angles, may further comprise diffraction peaks at one or more of the following positions: 18.04±0.20°, 18.43±0.20°, 22.94±0.20°, and 26.01±0.20°;
preferably, the X-ray powder diffraction pattern of the crystal form I, as determined by using Cu-Kα radiation and expressed in terms of 20 angles, comprises diffraction peaks at 5.67±0.20°, 11.37±0.20°, 12.01±0.20°, 13.89±0.20°, 16.69±0.20°, 17.05±0.20°, 17.32±0.20°, 18.04±0.20°, 18.43±0.20°, 19.42±0.20°, 19.73 ±0.20°, 22.94±0.20°, 23.41±0.20°, 26.01±0.20°, and 27.86±0.20°;
more preferably, the X-ray powder diffraction pattern of the crystal form I, as determined by using Cu-Kα radiation and expressed in terms of 20 angles, comprises diffraction peaks at positions as shown in the following table:

| No. | Position in 2θ (°) | No. | Position in 2θ (°) |
|---|---|---|---|
| 1 | 3.50 | 23 | 22.94 |
| 2 | 5.67 | 24 | 23.41 |
| 3 | 6.88 | 25 | 24.03 |
| 4 | 9.13 | 26 | 24.23 |
| 5 | 11.37 | 27 | 24.45 |
| 6 | 12.01 | 28 | 25.00 |
| 7 | 12.37 | 29 | 25.45 |
| 8 | 13.89 | 30 | 26.01 |
| 9 | 14.20 | 31 | 27.19 |
| 10 | 15.05 | 32 | 27.36 |
| 11 | 16.69 | 33 | 27.86 |
| 12 | 17.05 | 34 | 28.76 |
| 13 | 17.32 | 35 | 29.57 |
| 14 | 18.04 | 36 | 31.29 |
| 15 | 18.43 | 37 | 33.18 |
| 16 | 18.69 | 38 | 33.62 |
| 17 | 19.42 | 39 | 35.40 |
| 18 | 19.73 | 40 | 35.87 |
| 19 | 20.98 | 41 | 36.75 |
| 20 | 21.17 | 42 | 38.23 |

(continued)

| No. | Position in 2θ (°) | No. | Position in 2θ (°) |
|---|---|---|---|
| 21 | 21.80 | 43 | 39.71 |
| 22 | 22.32 | | |

;

most preferably, the X-ray powder diffraction pattern of the crystal form I, as determined by using Cu-Kα radiation and expressed in terms of 2θ angles, comprises diffraction peaks with positions and relative intensities as shown in the following table:

| No. | Position in 20 (°) | Relative intensity | No. | Position in 2θ (°) | Relative intensity |
|---|---|---|---|---|---|
| 1 | 3.50 | 3.00% | 23 | 22.94 | 10.60% |
| 2 | 5.67 | 45.80% | 24 | 23.41 | 25.50% |
| 3 | 6.88 | 3.00% | 25 | 24.03 | 3.40% |
| 4 | 9.13 | 6.90% | 26 | 24.23 | 5.90% |
| 5 | 11.37 | 38.70% | 27 | 24.45 | 3.40% |
| 6 | 12.01 | 25.90% | 28 | 25.00 | 1.80% |
| 7 | 12.37 | 6.50% | 29 | 25.45 | 2.30% |
| 8 | 13.89 | 11.90% | 30 | 26.01 | 10.40% |
| 9 | 14.20 | 4.10% | 31 | 27.19 | 1.80% |
| 10 | 15.05 | 2.40% | 32 | 27.36 | 1.50% |
| 11 | 16.69 | 36.70% | 33 | 27.86 | 15.00% |
| 12 | 17.05 | 25.60% | 34 | 28.76 | 2.80% |
| 13 | 17.32 | 28.90% | 35 | 29.57 | 4.70% |
| 14 | 18.04 | 10.70% | 36 | 31.29 | 3.40% |
| 15 | 18.43 | 10.30% | 37 | 33.18 | 0.90% |
| 16 | 18.69 | 5.00% | 38 | 33.62 | 2.00% |
| 17 | 19.42 | 13.70% | 39 | 35.40 | 0.90% |
| 18 | 19.73 | 100.00% | 40 | 35.87 | 1.60% |
| 19 | 20.98 | 2.30% | 41 | 36.75 | 0.60% |
| 20 | 21.17 | 6.30% | 42 | 38.23 | 0.60% |
| 21 | 21.80 | 1.40% | 43 | 39.71 | 1.00% |
| 22 | 22.32 | 3.60% | | | |

;

(2) the crystal form I has a differential scanning calorimetry pattern comprising an endothermic peak at 231.1 ±2°C; and

(3) the crystal form I has a thermogravimetric analysis pattern showing no weight loss before 200+2°C.

**3.** The crystal form I according to claim 2, wherein the crystal form I satisfies one or more of the following conditions:

(1) the X-ray powder diffraction pattern of the crystal form I, as determined by using Cu-Kα radiation and expressed in terms of 20 angles, is substantially as shown in FIG. 2;

(2) the differential scanning calorimetry pattern of the crystal form I is substantially as shown in FIG. 3; and

(3) the thermogravimetric analysis pattern of the crystal form I is substantially as shown in FIG. 3.

**4.** A crystal form II of a nitrogen-containing spiro compound of formula A, wherein the crystal form II has an X-ray powder diffraction pattern comprising diffraction peaks at 11.32± 0.20°, 11.70±0.20°, 11.93±0.20°, 18.03±0.20°, 18.81 ±0.20°, and 19.17±0.20°, as determined by using Cu-Kα radiation and expressed in terms of 2θ angles;

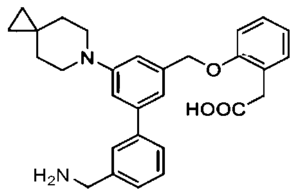

A

.

**5.** The crystal form II according to claim 4, wherein the crystal form II satisfies one or more of the following conditions:

(1) the X-ray powder diffraction pattern of the crystal form II, as determined by using Cu-Kα radiation and expressed in terms of 20 angles, further comprises diffraction peaks at one or more of the following positions: 5.54 ±0.20°, 12.37±0.20°, 13.78±0.20°, 14.11±0.20°, 15.78±0.20°, and 20.80±0.20°;

the X-ray powder diffraction pattern of the crystal form II, as determined by using Cu-Kα radiation and expressed in terms of 20 angles, may further comprise diffraction peaks at one or more of the following positions: 13.55± 0.20°, 16.40±0.20°, 17.74±0.20°, 20.49±0.20°, and 22.06±0.20°;
preferably, the X-ray powder diffraction pattern of the crystal form II, as determined by using Cu-Kα radiation and expressed in terms of 2θ angles, comprises diffraction peaks at 5.54±0.20°, 11.32±0.20°, 11.70±0.20°, 11.93±0.20°, 12.37±0.20°, 13.55±0.20°, 13.78±0.20°, 14.11+0.20°, 15.78±0.20°, 16.40+0.20°, 17.74 ±0.20°, 18.03+0.20°, 18.81±0.20°, 19.17±0.20°, 20.49±0.20°, 20.80±0.20°, and 22.06±0.20°;
more preferably, the X-ray powder diffraction pattern of the crystal form II, as determined by using Cu-Kα radiation and expressed in terms of 20 angles, comprises diffraction peaks at positions as shown in the following table:

| No. | Position in 20 (°) | No. | Position in 2θ (°) |
|---|---|---|---|
| 1 | 5.54 | 23 | 19.17 |
| 2 | 7.20 | 24 | 19.68 |
| 3 | 8.63 | 25 | 20.21 |
| 4 | 9.04 | 26 | 20.49 |
| 5 | 11.32 | 27 | 20.80 |
| 6 | 11.70 | 28 | 21.23 |
| 7 | 11.93 | 29 | 22.06 |
| 8 | 12.37 | 30 | 22.52 |
| 9 | 12.80 | 31 | 23.15 |
| 10 | 13.55 | 32 | 23.54 |
| 11 | 13.78 | 33 | 24.44 |
| 12 | 14.11 | 34 | 24.78 |
| 13 | 14.52 | 35 | 25.33 |
| 14 | 15.23 | 36 | 25.92 |
| 15 | 15.78 | 37 | 26.40 |
| 16 | 16.40 | 38 | 26.68 |
| 17 | 16.92 | 39 | 27.52 |
| 18 | 17.31 | 40 | 28.36 |
| 19 | 17.74 | 41 | 29.62 |

(continued)

| No. | Position in 2θ (°) | No. | Position in 2θ (°) |
|---|---|---|---|
| 20 | 18.03 | 42 | 30.06 |
| 21 | 18.41 | 43 | 31.61 |
| 22 | 18.81 | 44 | 34.52 |

most preferably, the X-ray powder diffraction pattern of the crystal form II, as determined by using Cu-Kα radiation and expressed in terms of 2θ angles, comprises diffraction peaks with positions and relative intensities as shown in the following table:

| No. | Position in 2θ (°) | Relative intensity | No. | Position in 2θ (°) | Relative intensity |
|---|---|---|---|---|---|
| 1 | 5.54 | 62.50% | 23 | 19.17 | 100.00% |
| 2 | 7.20 | 4.60% | 24 | 19.68 | 31.90% |
| 3 | 8.63 | 19.60% | 25 | 20.21 | 11.00% |
| 4 | 9.04 | 15.80% | 26 | 20.49 | 48.80% |
| 5 | 11.32 | 64.40% | 27 | 20.80 | 51.00% |
| 6 | 11.70 | 65.00% | 28 | 21.23 | 10.40% |
| 7 | 11.93 | 92.80% | 29 | 22.06 | 50.40% |
| 8 | 12.37 | 64.30% | 30 | 22.52 | 34.30% |
| 9 | 12.80 | 29.20% | 31 | 23.15 | 27.40% |
| 10 | 13.55 | 37.70% | 32 | 23.54 | 16.90% |
| 11 | 13.78 | 57.00% | 33 | 24.44 | 13.00% |
| 12 | 14.11 | 54.40% | 34 | 24.78 | 19.60% |
| 13 | 14.52 | 36.10% | 35 | 25.33 | 11.20% |
| 14 | 15.23 | 36.80% | 36 | 25.92 | 9.40% |
| 15 | 15.78 | 61.50% | 37 | 26.40 | 9.00% |
| 16 | 16.40 | 40.10% | 38 | 26.68 | 8.20% |
| 17 | 16.92 | 25.00% | 39 | 27.52 | 13.40% |
| 18 | 17.31 | 32.50% | 40 | 28.36 | 5.40% |
| 19 | 17.74 | 41.80% | 41 | 29.62 | 6.50% |
| 20 | 18.03 | 95.30% | 42 | 30.06 | 5.90% |
| 21 | 18.41 | 30.00% | 43 | 31.61 | 3.80% |
| 22 | 18.81 | 69.20% | 44 | 34.52 | 5.70% |

;

(2) the crystal form II has a differential scanning calorimetry pattern comprising an endothermic peak at 230.7 ±2°C; and

(3) the crystal form II has a differential scanning calorimetry pattern comprising an exothermic peak at 159.1 ±2°C; and

(4) the crystal form II has a thermogravimetric analysis pattern showing a weight loss of 1.71% before 210±2°C.

**6.** The crystal form II according to claim 5, wherein the crystal form II satisfies one or more of the following conditions:

(1) the X-ray powder diffraction pattern of the crystal form II, as determined by using Cu-Kα radiation and expressed in terms of 2θ angles, is substantially as shown in FIG. 6;

(2) the differential scanning calorimetry pattern of the crystal form II is substantially as shown in FIG. 7; and
(3) the thermogravimetric analysis pattern of the crystal form II is substantially as shown in FIG. 7.

**7.** A preparation method for the crystal form I as defined in any one of claims 1 to 3, wherein the preparation method comprises the following steps: dissolving the nitrogen-containing spiro compound of formula A in N-methylpyrrolidone, adding an antisolvent, and crystallizing; wherein the antisolvent is one or more selected from the group consisting of nitrile solvents, ester solvents, benzene solvents, ether solvents, and water;
preferably, the preparation method for the crystal form I satisfies one or more of the following conditions:

(1) the method for the dissolving is heating with stirring; the temperature for the heating may be 30 to 70°C, preferably 40 to 60°C, such as 50°C;
(2) the volume-to-mass ratio of N-methylpyrrolidone to the nitrogen-containing spiro compound of formula A is 15 to 50 mL/g, preferably 20 to 35 mL/g, such as 26.7 mL/g;
(3) the volume-to-mass ratio of the antisolvent to the nitrogen-containing spiro compound of formula A is 50 to 350 mL/g, preferably 80 to 250 mL/g, such as 80 mL/g, 160 mL/g, or 213.3 mL/g;
(4) the volume ratio of the antisolvent to N-methylpyrrolidone is (1 to 10):1, preferably (3 to 8):1, such as 3:1, 6:1, or 8:1;
(5) a cooling step is further included before adding the antisolvent; the cooling step may involve cooling to between 10°C and 20°C, such as 17°C; the cooling step may have a cooling rate of 0.2°C/min;
(6) the nitrile solvent is acetonitrile;
(7) the ester solvent is one or more of ethyl acetate, n-propyl acetate, and isopropyl acetate, such as ethyl acetate and/or isopropyl acetate;
(7) the benzene solvent is one or more of toluene, ethylbenzene, xylene, isopropylbenzene, and chlorobenzene, such as toluene; and
(8) the ether solvent is one or more of methyl *tert*-butyl ether, diethyl ether, cyclopentyl methyl ether, isopropyl ether, methyl tetrahydrofuran, and dioxane, such as methyl tert-butyl ether.

**8.** A preparation method for the crystal form II as defined in any one of claims 4 to 6, wherein the preparation method comprises the following steps: stirring the nitrogen-containing spiro compound of formula A in acetonitrile for 12 to 16 days;
preferably, the preparation method for the crystal form II satisfies one or more of the following conditions:

(1) the volume-to-mass ratio of acetonitrile to the nitrogen-containing spiro compound of formula A is 15 to 50 mL/g, preferably 25 to 40 mL/g, such as 33.3 mL/g;
(2) the temperature for the stirring is 15 to 30°C, such as 17°C; and
(3) the time for the stirring is 14 days.

**9.** A pharmaceutical composition comprising: (1) the crystal form I as defined in any one of claims 1 to 3 or the crystal form II as defined in any one of claims 4 to 6; and
(2) a pharmaceutically acceptable carrier.

**10.** A use of the crystal form I as defined in any one of claims 1 to 3 or crystal form II as defined in any one of claims 4 to 6 in the manufacture of a medicament for treating and/or preventing a complement factor D-mediated disease, wherein the complement factor D-mediated disease is a blood disease, kidney disease, cardiovascular disease, immune disorder, central nervous system disease, respiratory disease, urogenital disease, or ocular disease;
preferably, the complement factor D-mediated disease is cold agglutinin disease, catastrophic antiphospholipid syndrome, hemolytic anemia, antineutrophil cytoplasmic antibody-associated vasculitis, warm autoimmune hemolytic anemia, paroxysmal nocturnal hemoglobinuria, IgA nephropathy, lupus nephritis, atypical hemolytic uremic syndrome, membranoproliferative glomerulonephritis, dense deposit disease, C3 glomerulonephritis, focal segmental glomerulosclerosis, diabetic nephropathy, systemic lupus erythematosus or lupus erythematosus, rheumatoid arthritis, inflammatory bowel disease, psoriasis, multiple sclerosis, organ transplant rejection, myasthenia gravis, Alzheimer's disease, respiratory distress syndrome, asthma, chronic obstructive pulmonary disease, emphysema, coronavirus infection, macular degeneration, age-related macular degeneration, macular edema, diabetic macular edema, choroidal neovascularization, uveitis, Behçet's uveitis, proliferative diabetic retinopathy, non-proliferative diabetic retinopathy, glaucoma, hypertensive retinopathy, corneal neovascularization, corneal transplant rejection, corneal dystrophy, autoimmune dry eye disease, Stevens-Johnson syndrome, Sjögren's syndrome, environmental dry eye disease, Fuchs' endothelial corneal dystrophy, retinal vein occlusion, or postoperative inflammation; the coronavirus infection may be SARS-CoV, MERS-CoV, or SARS-CoV-2 infection.

**11.** A use of the crystal form I as defined in any one of claims 1 to 3 or the crystal form II as defined in any one of claims 4 to 6 in the manufacture of a medicament for treating and/or preventing a disease, wherein the disease is a blood disease, kidney disease, cardiovascular disease, immune disorder, central nervous system disease, respiratory disease, urogenital disease, or ocular disease;
preferably, the disease is cold agglutinin disease, catastrophic antiphospholipid syndrome, hemolytic anemia, anti-neutrophil cytoplasmic antibody-associated vasculitis, warm autoimmune hemolytic anemia, paroxysmal nocturnal hemoglobinuria, IgA nephropathy, lupus nephritis, atypical hemolytic uremic syndrome, membranoproliferative glomerulonephritis, dense deposit disease, C3 glomerulonephritis, focal segmental glomerulosclerosis, diabetic nephropathy, systemic lupus erythematosus or lupus erythematosus, rheumatoid arthritis, inflammatory bowel disease, psoriasis, multiple sclerosis, organ transplant rejection, myasthenia gravis, Alzheimer's disease, respiratory distress syndrome, asthma, chronic obstructive pulmonary disease, emphysema, coronavirus infection, macular degeneration, age-related macular degeneration, macular edema, diabetic macular edema, choroidal neovascularization, uveitis, Behçet's uveitis, proliferative diabetic retinopathy, non-proliferative diabetic retinopathy, glaucoma, hypertensive retinopathy, corneal neovascularization, corneal transplant rejection, corneal dystrophy, autoimmune dry eye disease, Stevens-Johnson syndrome, Sjögren's syndrome, environmental dry eye disease, Fuchs' endothelial corneal dystrophy, retinal vein occlusion, or postoperative inflammation; the coronavirus infectio may be SARS-CoV, MERS-CoV, or SARS-CoV-2 infection.

**12.** A use of the crystal form I as defined in any one of claims 1 to 3 or the crystal form II as defined in any one of claims 4 to 6 in the manufacture of a complement factor D inhibitor;
the complement factor D inhibitor may be used *in vivo* or *in vitro* in mammals.

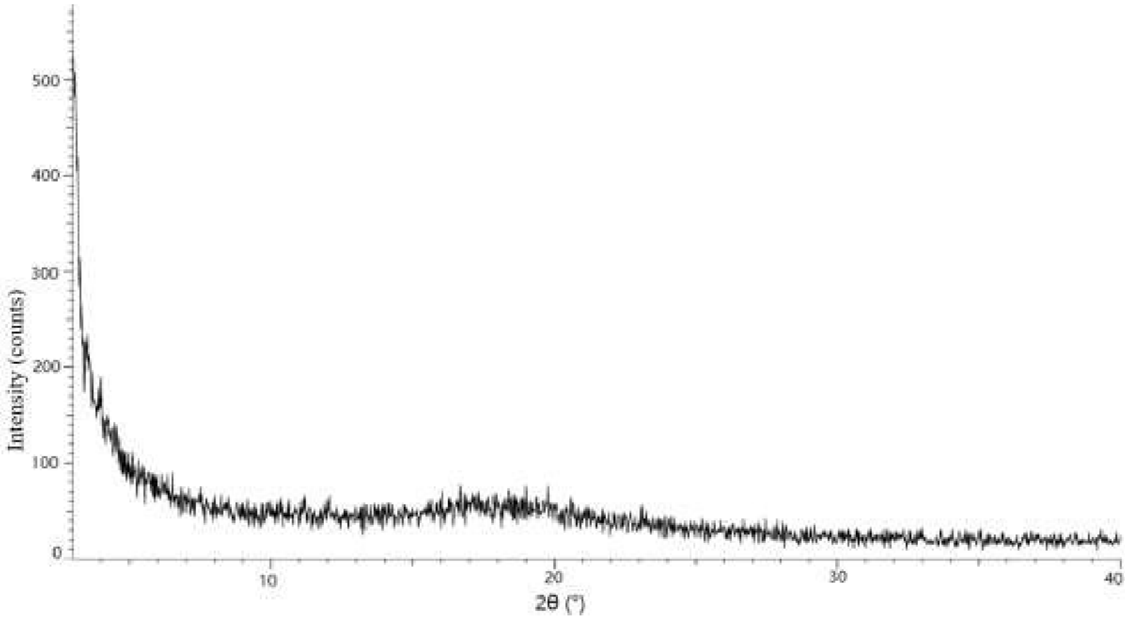


FIG. 1

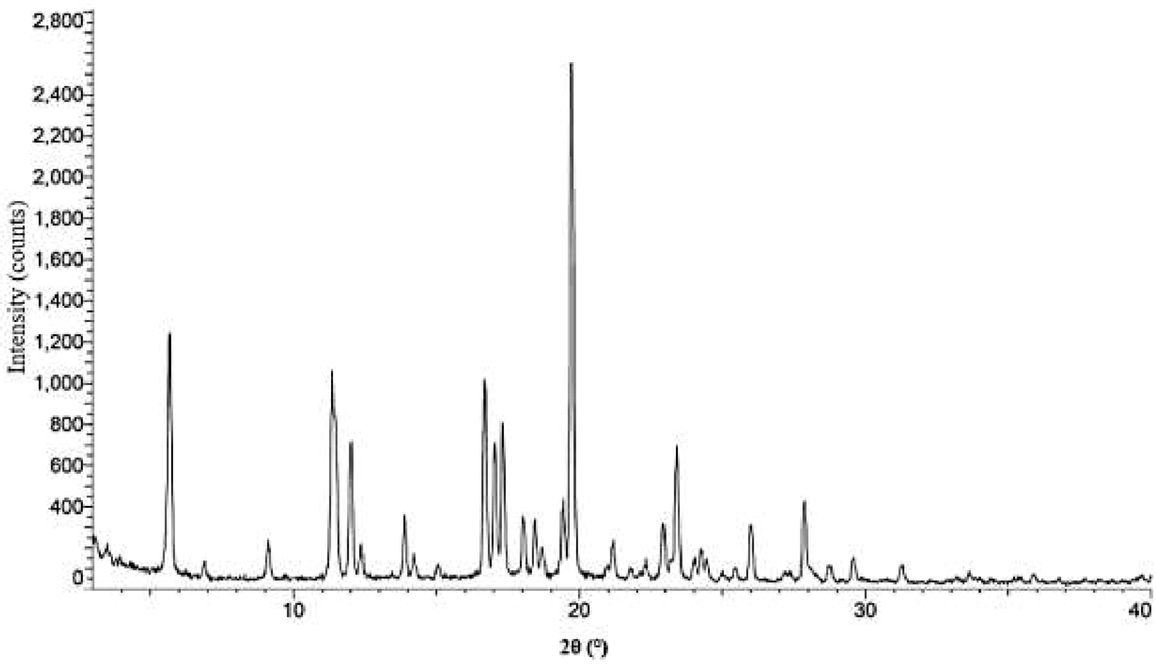
2,800
2,400
2,200
2,000
1,800
1,600
1,400
1,200
1,000
800
600
400
0
Intensity (counts)
10
20
30
40
2θ (°)

FIG. 2

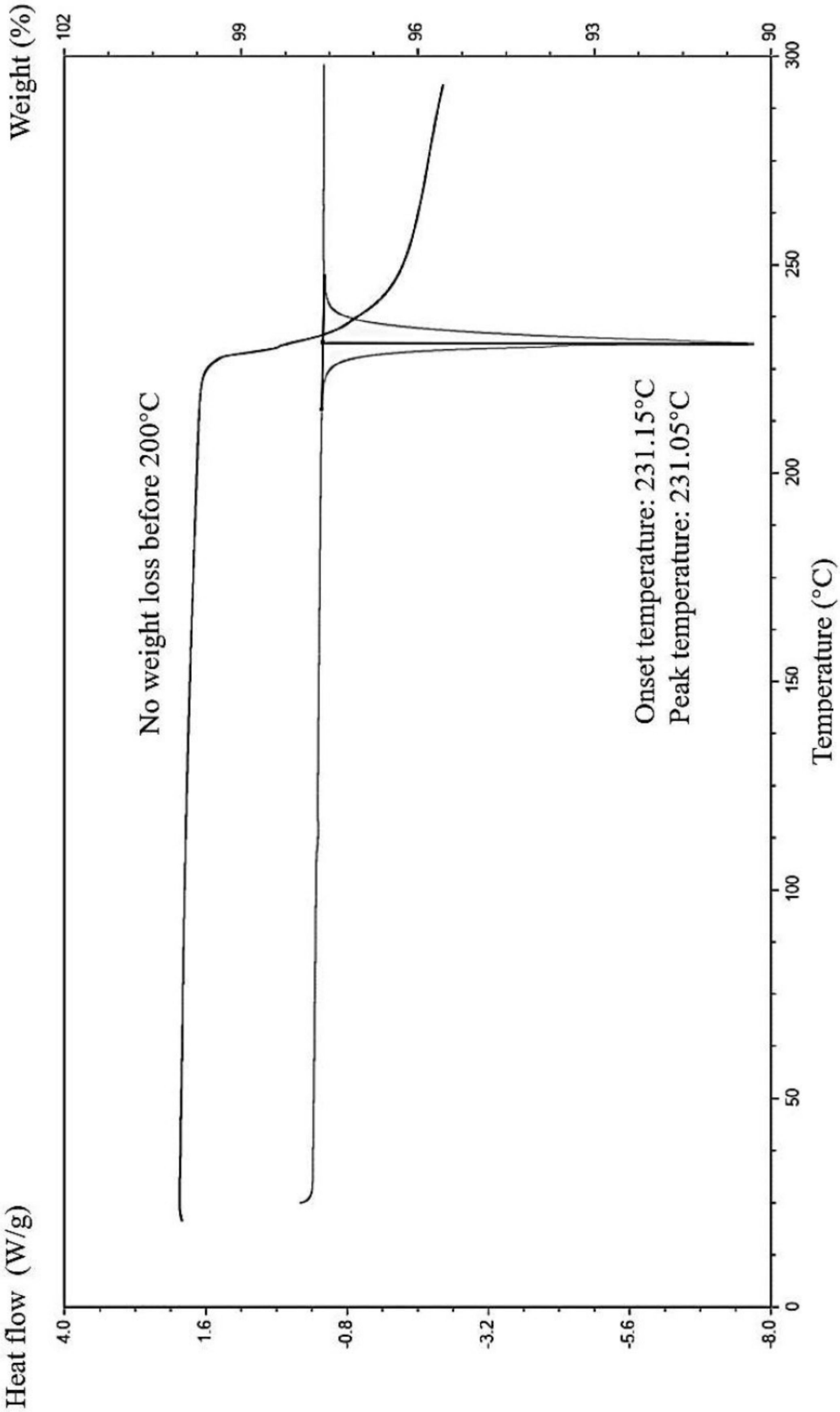

Weight (%)
102
99
96
93
90
Heat flow (W/g)
4.0
1.6
-0.8
-3.2
-5.6
-8.0
0
50
100
150
200
250
300
No weight loss before 200°C
Onset temperature: 231.15°C
Peak temperature: 231.05°C
Temperature (°C)


FIG. 3

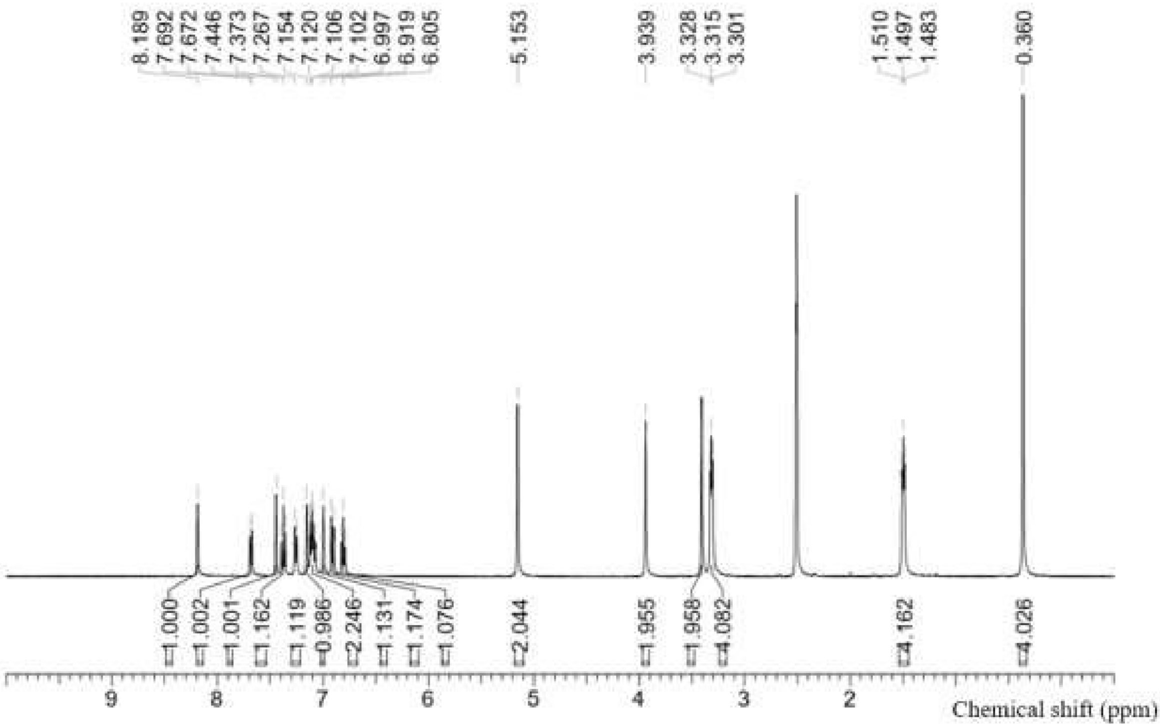
8.189
7.692
7.672
7.446
7.373
7.267
7.154
7.120
7.106
7.102
6.997
6.919
6.805
5.153
3.939
3.328
3.315
3.301
1.510
1.497
1.483
0.360
1.000
1.002
1.001
1.162
1.119
0.986
2.246
1.131
1.174
1.076
2.044
1.955
1.958
4.082
4.162
4.026
9
8
7
6
5
4
3
2
Chemical shift (ppm)

FIG. 4

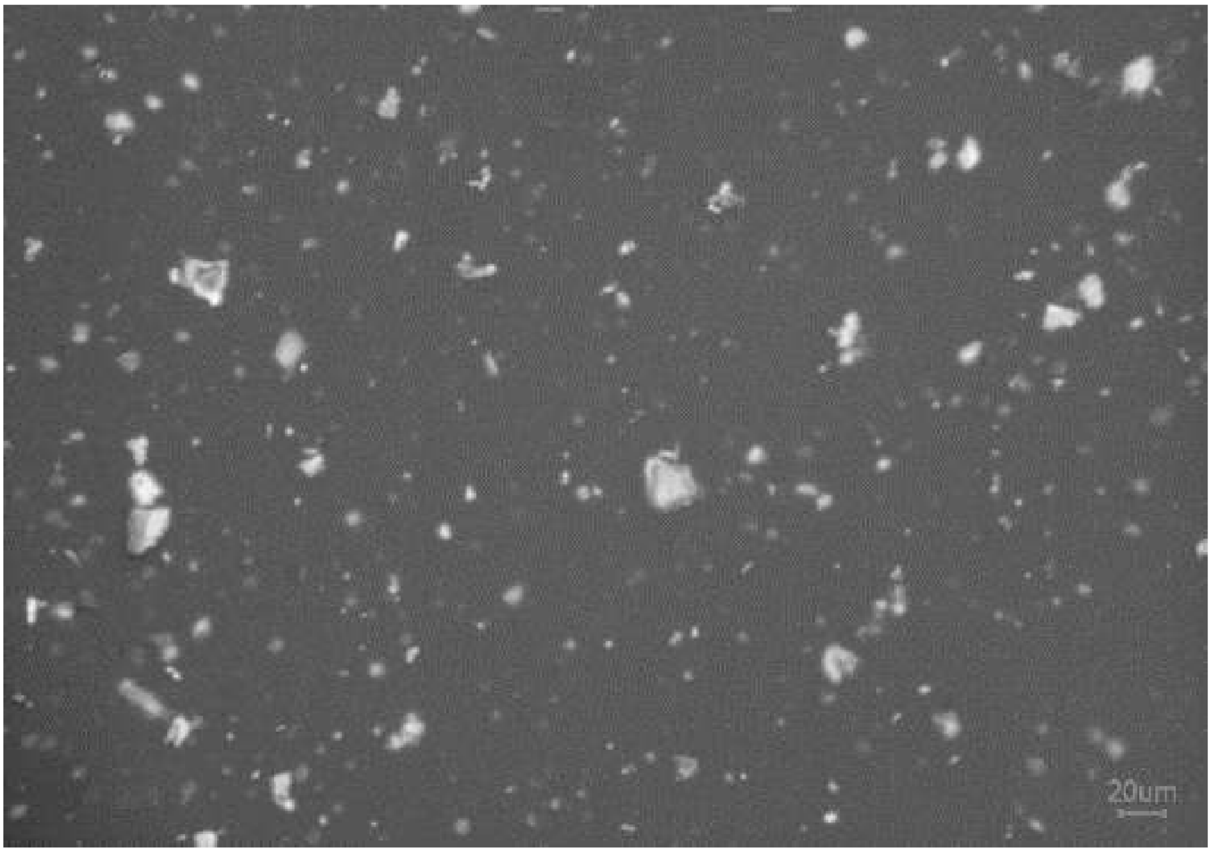
20um

FIG. 5

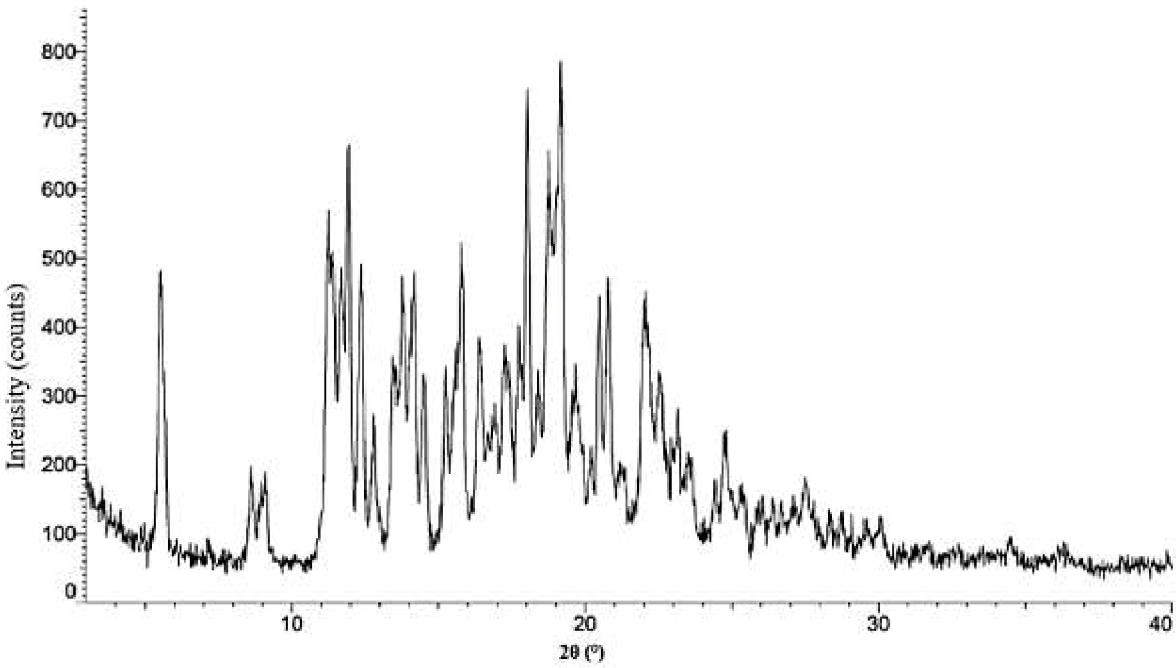

800
700
600
500
400
300
200
100
0
Intensity (counts)
10
20
30
40
2θ (°)


FIG. 6

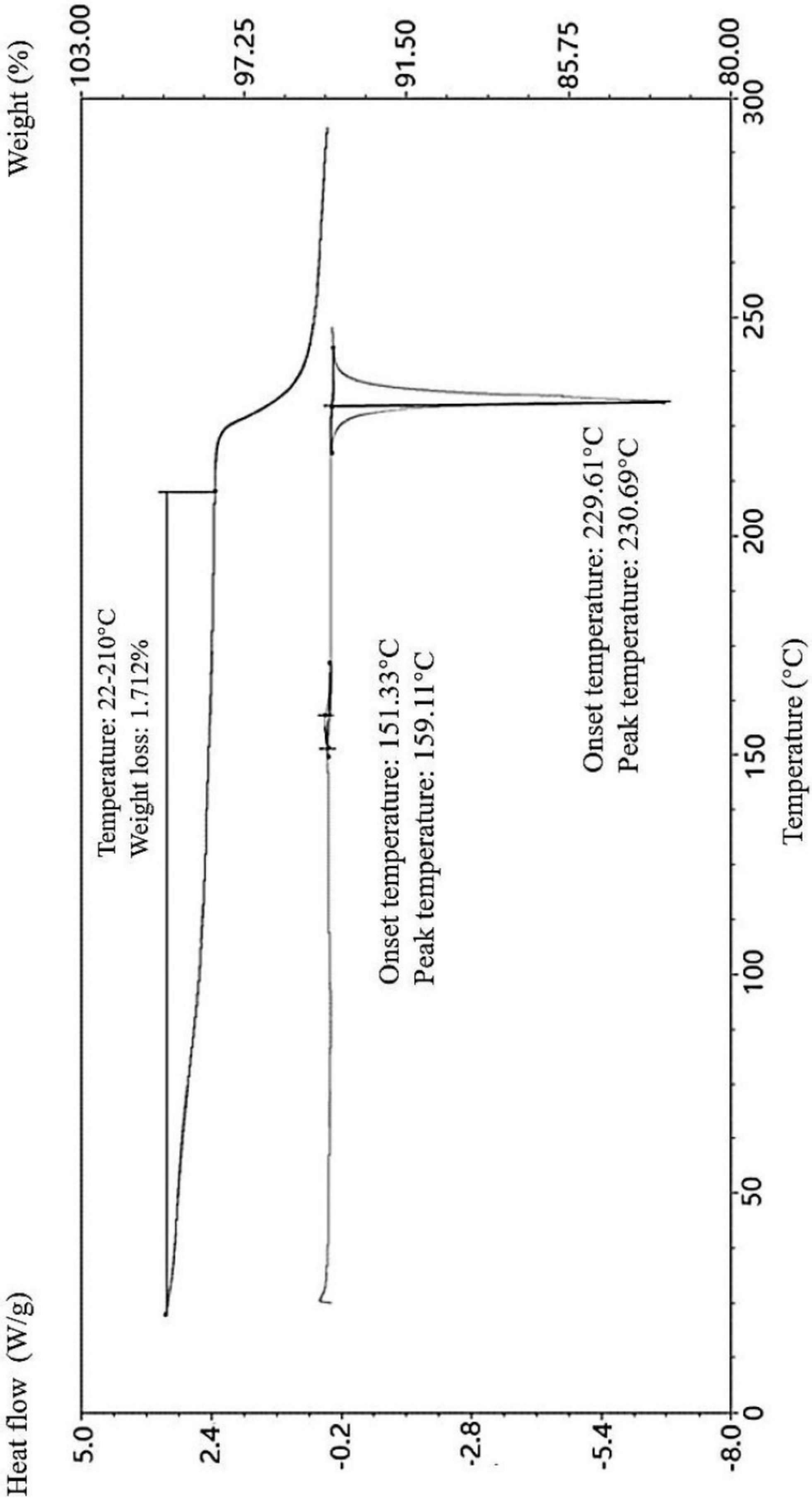
Weight (%)
103.00
97.25
91.50
85.75
80.00
Heat flow (W/g)
5.0
2.4
-0.2
-2.8
-5.4
-8.0
Temperature: 22-210°C
Weight loss: 1.712%
Onset temperature: 151.33°C
Peak temperature: 159.11°C
Onset temperature: 229.61°C
Peak temperature: 230.69°C
0
50
100
150
200
250
300
Temperature (°C)

FIG. 7

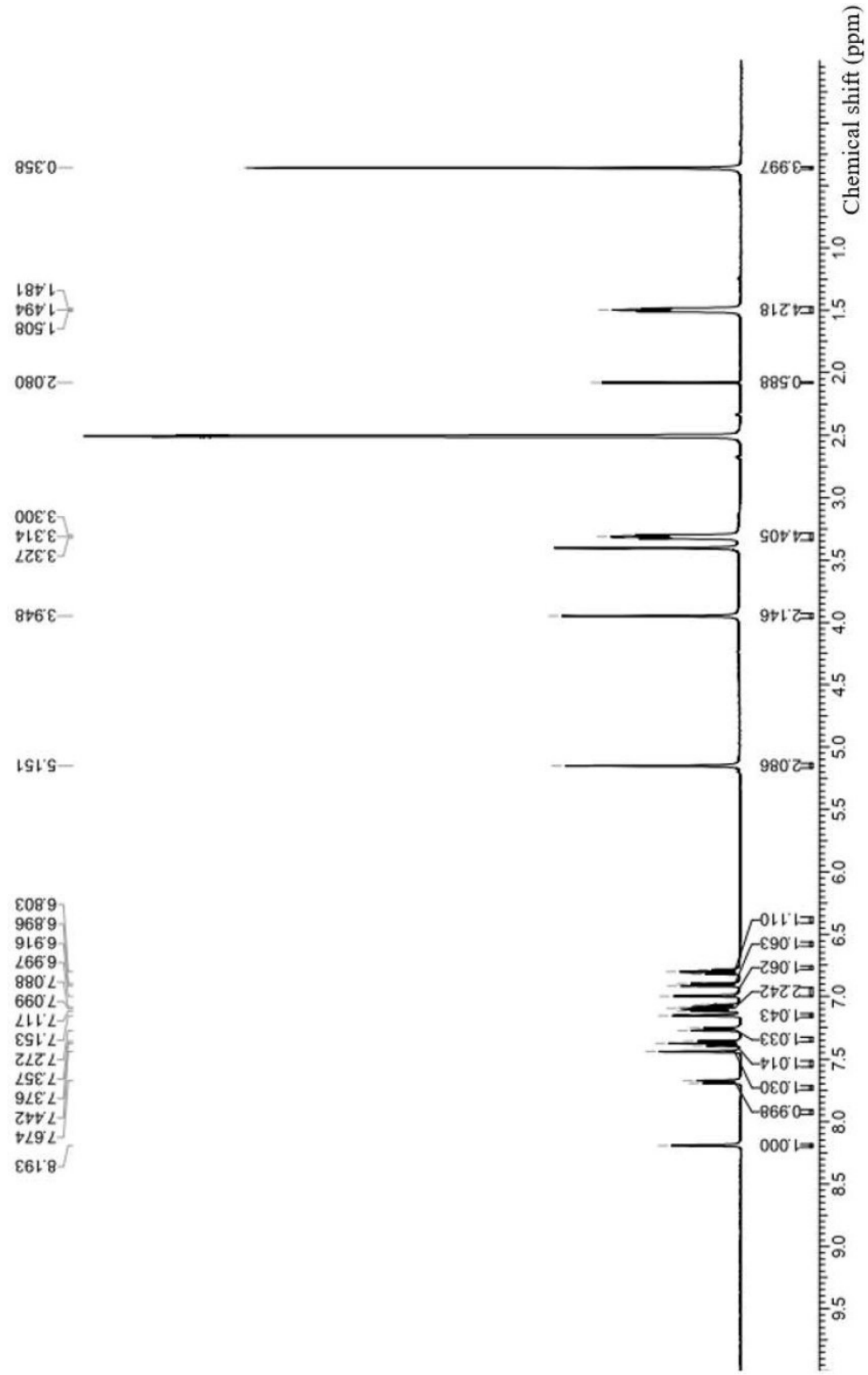
0.358
1.481
1.494
1.508
2.080
3.300
3.314
3.327
3.948
5.151
6.803
6.896
6.916
6.997
7.088
7.099
7.117
7.153
7.272
7.357
7.376
7.442
7.674
8.193
3.997
4.218
0.588
4.405
2.146
2.086
1.110
1.063
1.062
2.242
1.043
1.033
1.014
1.030
0.998
1.000
1.0
1.5
2.0
2.5
3.0
3.5
4.0
4.5
5.0
5.5
6.0
6.5
7.0
7.5
8.0
8.5
9.0
9.5
Chemical shift (ppm)

FIG. 8

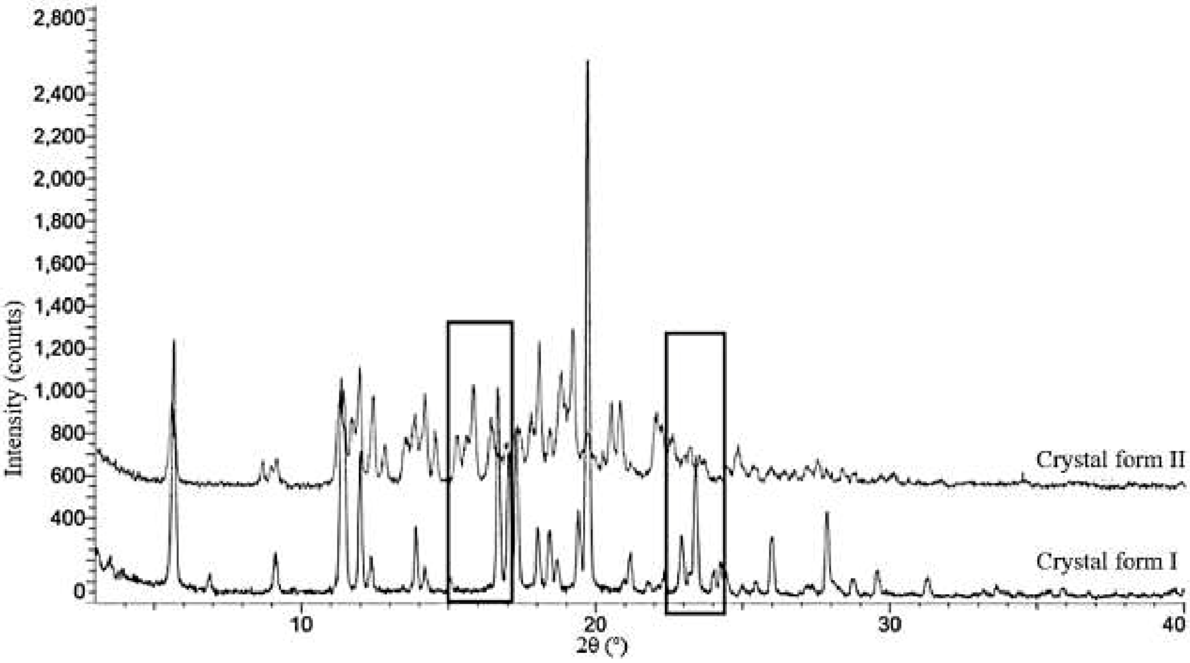
2,800
2,400
2,200
2,000
1,800
1,600
1,400
1,200
1,000
800
600
400
0
Intensity (counts)
Crystal form II
Crystal form I
10
20
30
40
2θ (°)

FIG. 9

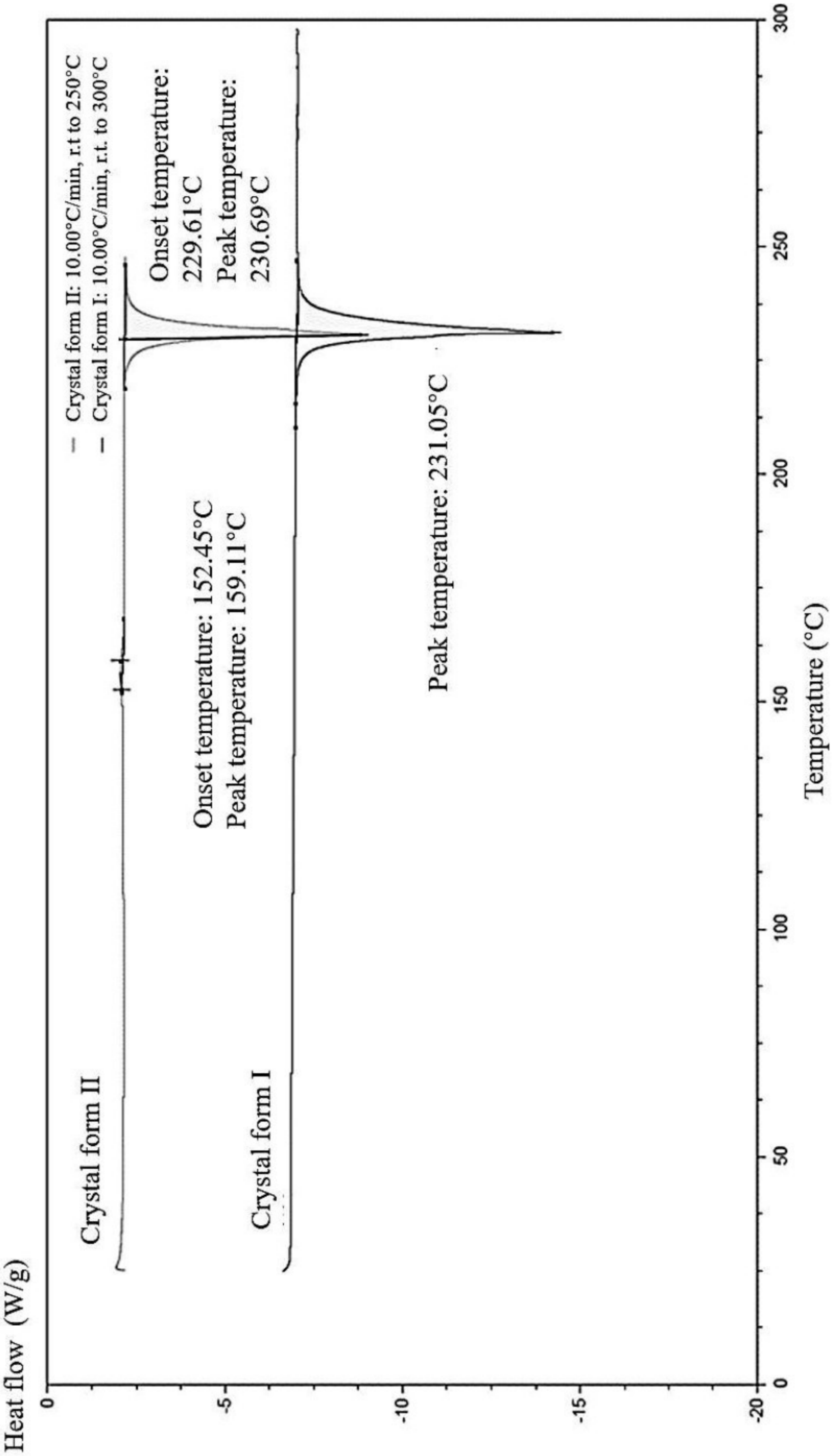
Heat flow (W/g)
Temperature (°C)
Crystal form II: 10.00°C/min, r.t to 250°C
Crystal form I: 10.00°C/min, r.t. to 300°C
Crystal form II
Crystal form I
Onset temperature: 152.45°C
Peak temperature: 159.11°C
Onset temperature: 229.61°C
Peak temperature: 230.69°C
Peak temperature: 231.05°C
0
-5
-10
-15
-20
0
50
100
150
200
250
300

FIG. 10

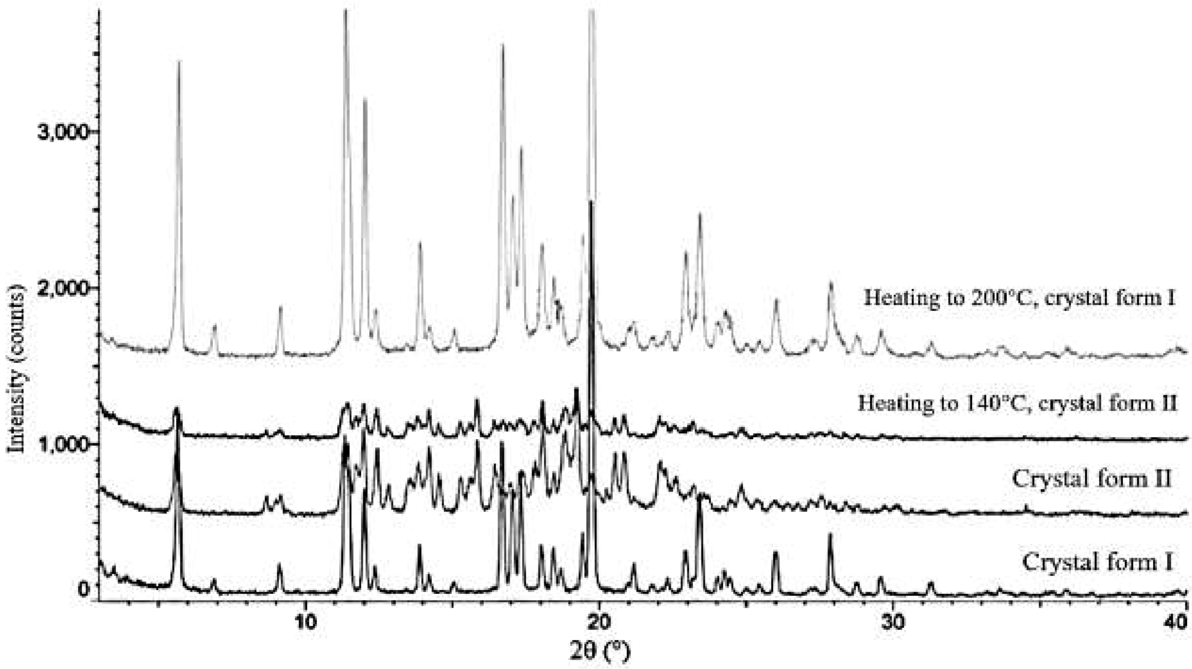
Intensity (counts)
2θ (°)
Heating to 200°C, crystal form I
Heating to 140°C, crystal form II
Crystal form II
Crystal form I

FIG. 11

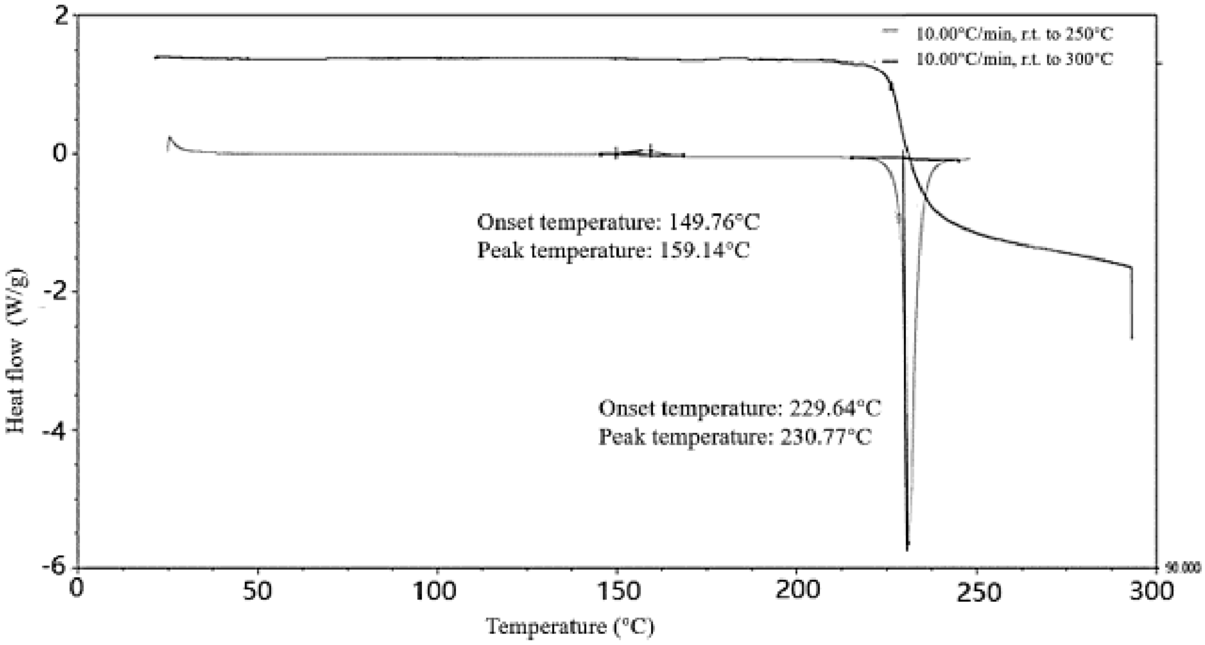
10.00°C/min, r.t. to 250°C
10.00°C/min, r.t. to 300°C
Onset temperature: 149.76°C
Peak temperature: 159.14°C
Onset temperature: 229.64°C
Peak temperature: 230.77°C
Heat flow (W/g)
Temperature (°C)

FIG. 12

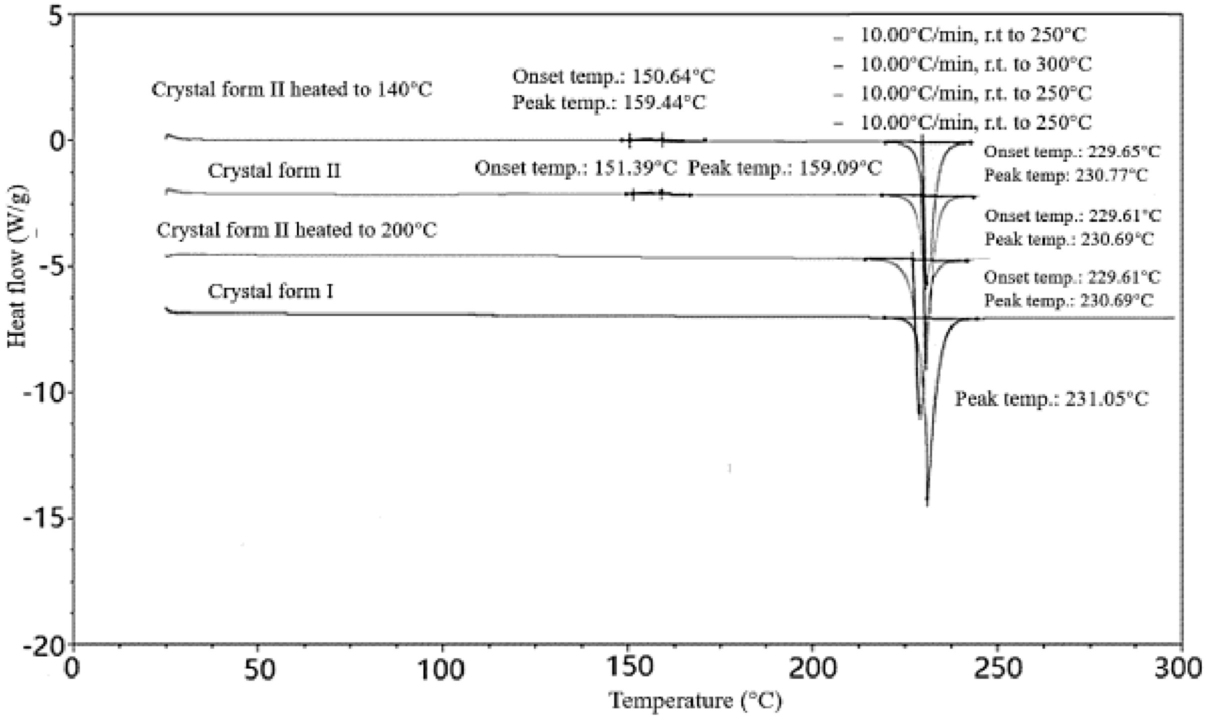
10.00°C/min, r.t to 250°C
10.00°C/min, r.t. to 300°C
10.00°C/min, r.t. to 250°C
10.00°C/min, r.t. to 250°C
Crystal form II heated to 140°C
Onset temp.: 150.64°C
Peak temp.: 159.44°C
Onset temp.: 229.65°C
Peak temp: 230.77°C
Crystal form II
Onset temp.: 151.39°C Peak temp.: 159.09°C
Onset temp.: 229.61°C
Peak temp.: 230.69°C
Crystal form II heated to 200°C
Onset temp.: 229.61°C
Peak temp.: 230.69°C
Crystal form I
Peak temp.: 231.05°C
Heat flow (W/g)
Temperature (°C)

FIG. 13

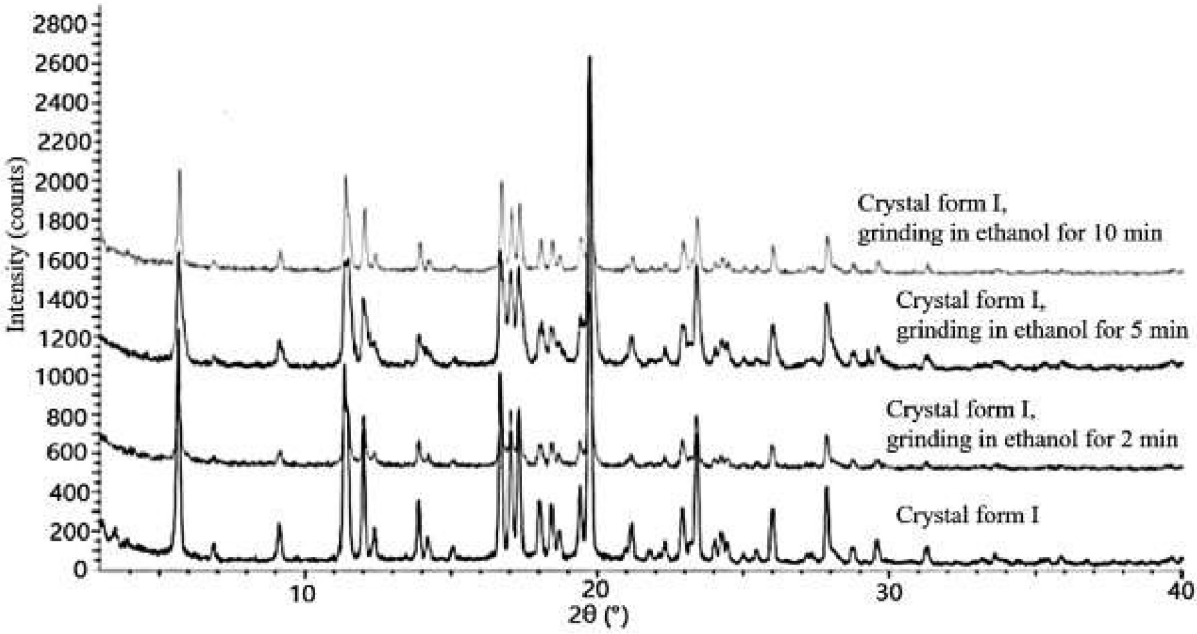
Crystal form I,
grinding in ethanol for 10 min
Crystal form I,
grinding in ethanol for 5 min
Crystal form I,
grinding in ethanol for 2 min
Crystal form I
Intensity (counts)
2θ (°)

FIG. 14

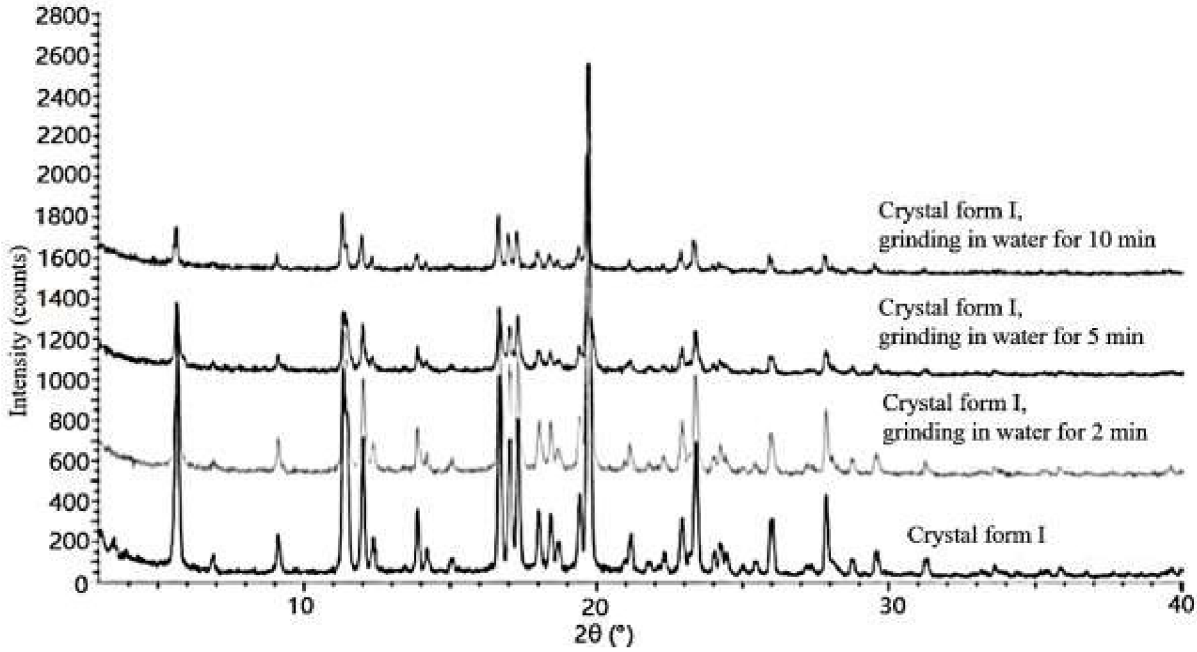
2800
2600
2400
2200
2000
1800
1600
1400
1200
1000
800
600
400
200
0
Intensity (counts)
Crystal form I,
grinding in water for 10 min
Crystal form I,
grinding in water for 5 min
Crystal form I,
grinding in water for 2 min
Crystal form I
10
20
30
40
2θ (°)

FIG. 15

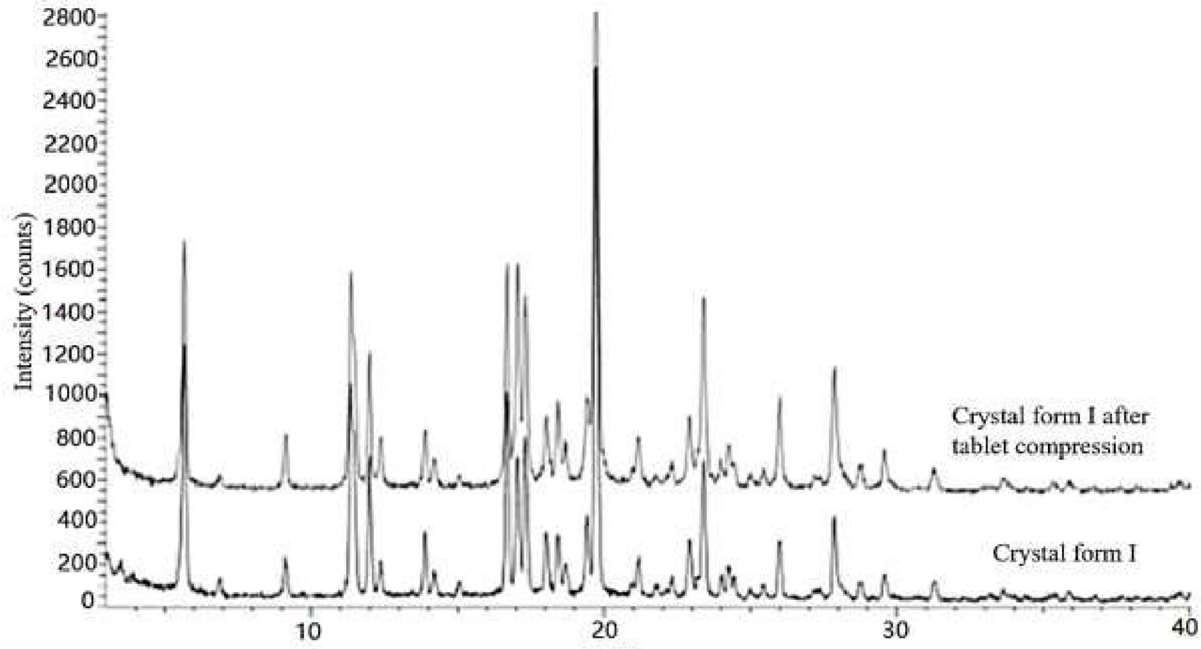
2800
2600
2400
2200
2000
1800
1600
1400
1200
1000
800
600
400
200
0
Intensity (counts)
Crystal form I after
tablet compression
Crystal form I
10
20
30
40
2θ (°)

FIG. 16

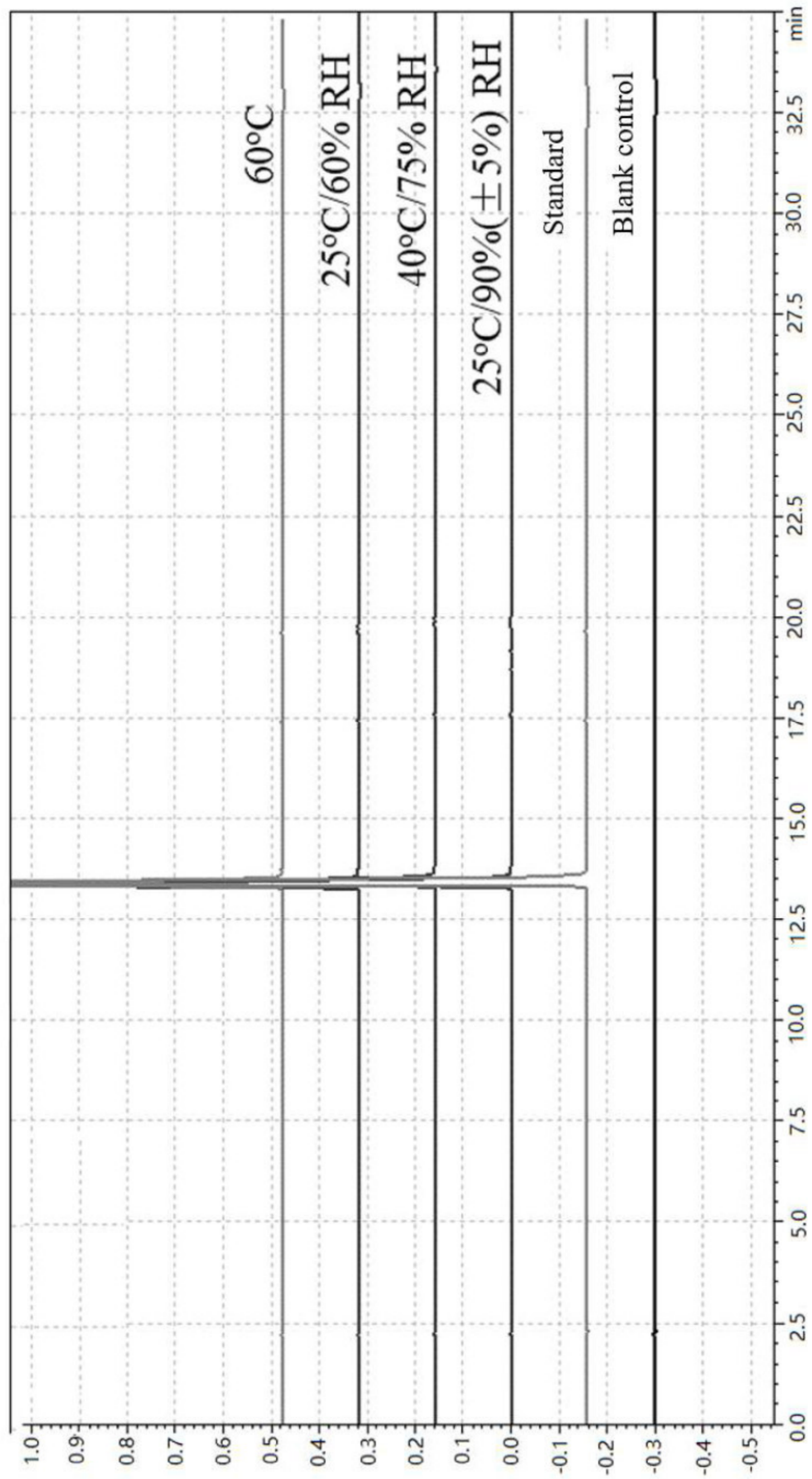


FIG. 17

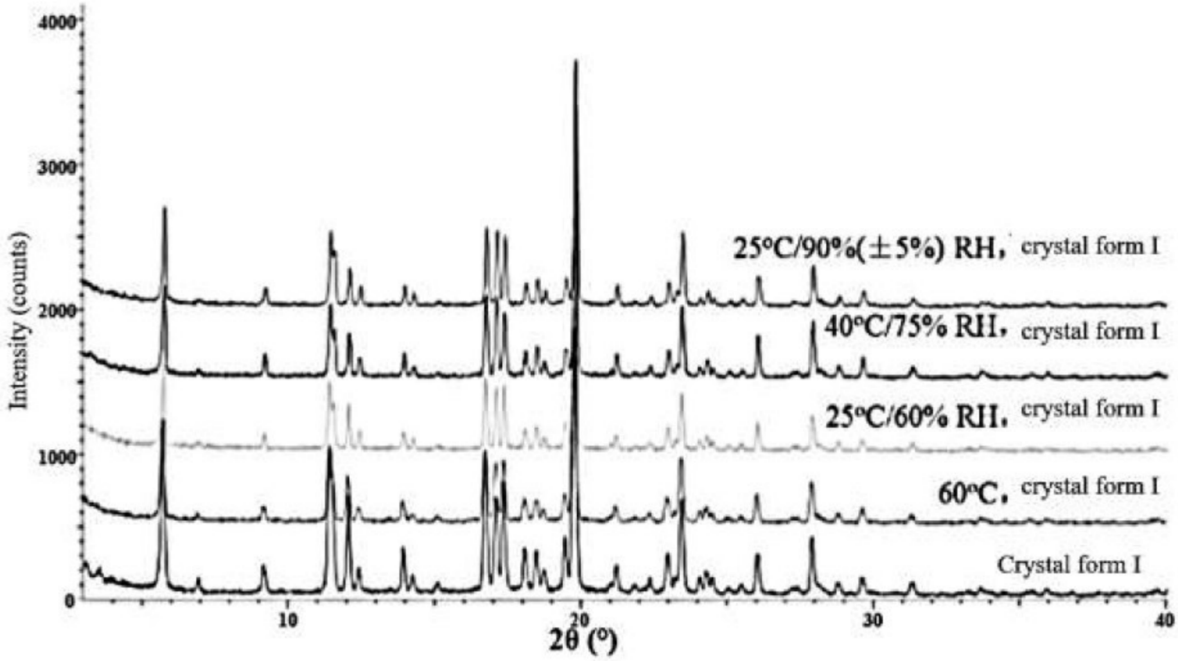
4000
3000
2000
1000
0
Intensity (counts)
10
20
30
40
2θ (°)
25°C/90%(±5%) RH, crystal form I
40°C/75% RH, crystal form I
25°C/60% RH, crystal form I
60°C, crystal form I
Crystal form I

FIG. 18

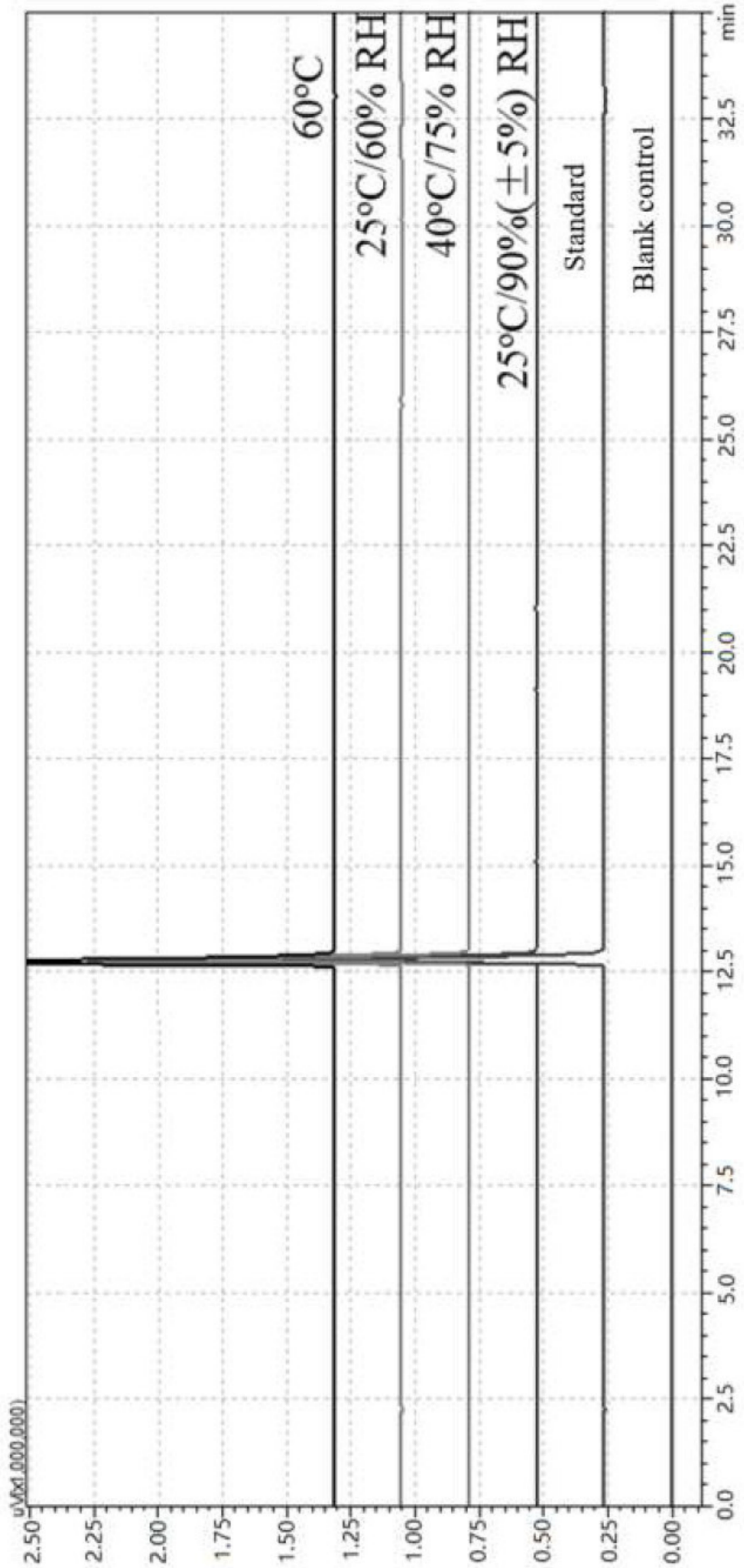
uVx1,000,000
60°C
25°C/60% RH
40°C/75% RH
25°C/90%(±5%) RH
Standard
Blank control
min
2.50
2.25
2.00
1.75
1.50
1.25
1.00
0.75
0.50
0.25
0.00
0.0
2.5
5.0
7.5
10.0
12.5
15.0
17.5
20.0
22.5
25.0
27.5
30.0
32.5

FIG. 19

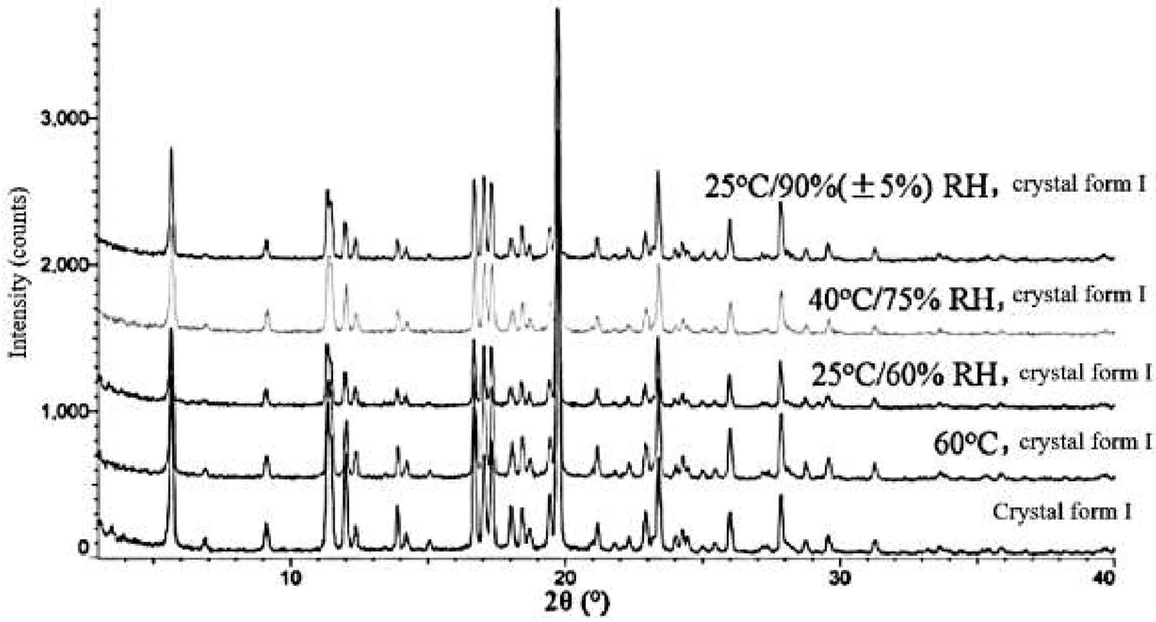

3,000
2,000
1,000
0
Intensity (counts)
10
20
30
40
2θ (°)
25°C/90%(±5%) RH, crystal form I
40°C/75% RH, crystal form I
25°C/60% RH, crystal form I
60°C, crystal form I
Crystal form I


FIG. 20

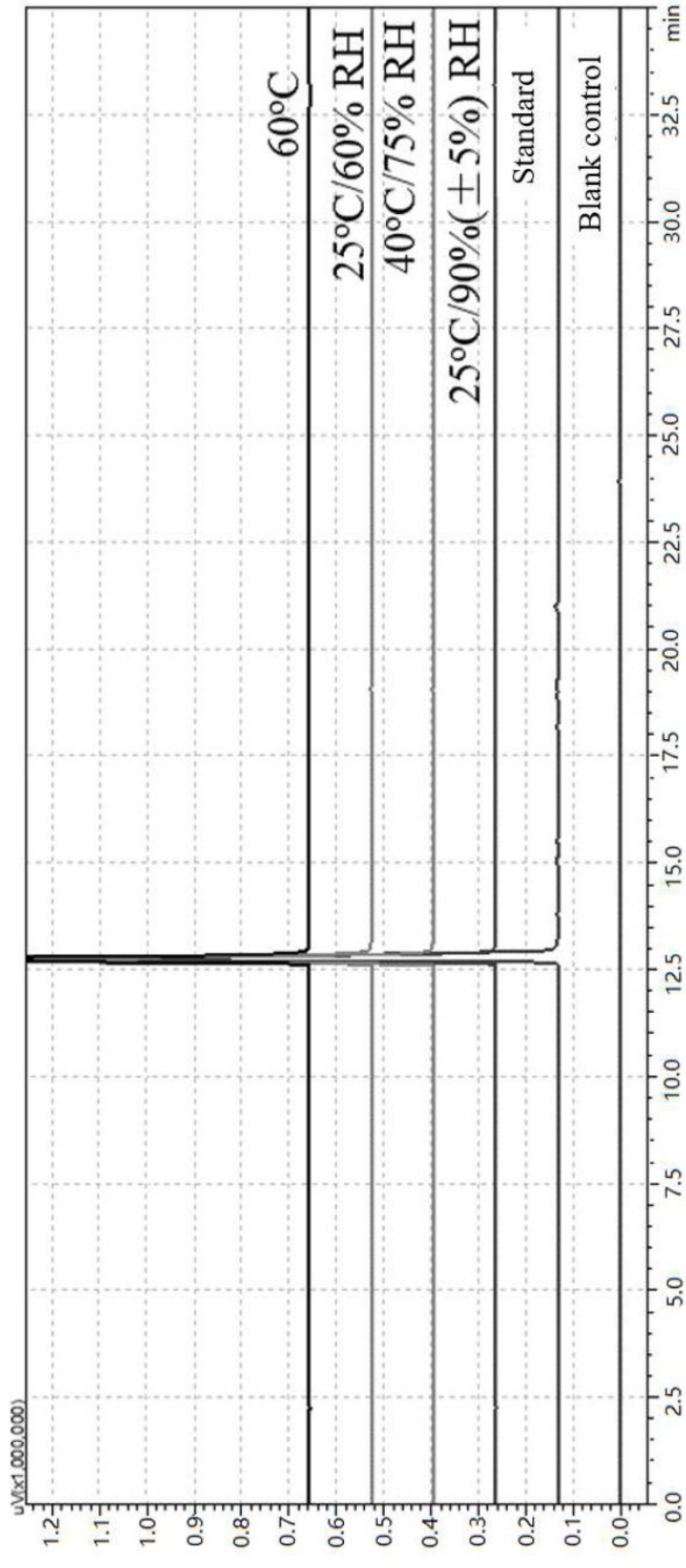
60°C
25°C/60% RH
40°C/75% RH
25°C/90%(±5%) RH
Standard
Blank control
uV(x1,000,000)
min

FIG. 21

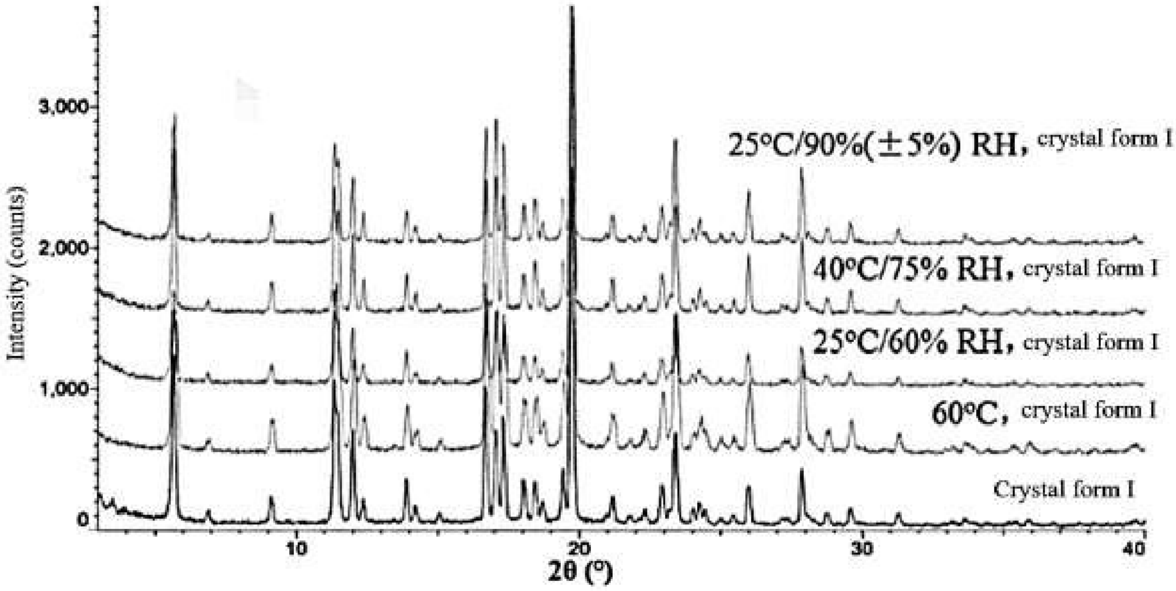
25°C/90%(±5%) RH, crystal form I
40°C/75% RH, crystal form I
25°C/60% RH, crystal form I
60°C, crystal form I
Crystal form I
Intensity (counts)
2θ (°)
0
1,000
2,000
3,000
10
20
30
40

FIG. 22

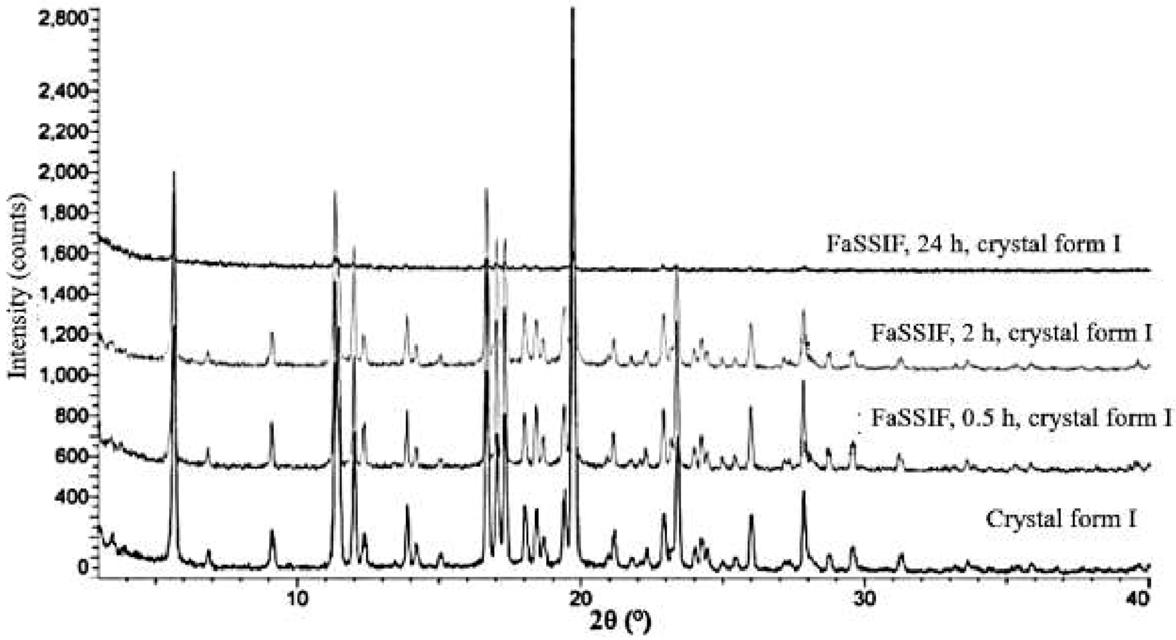
FaSSIF, 24 h, crystal form I
FaSSIF, 2 h, crystal form I
FaSSIF, 0.5 h, crystal form I
Crystal form I
Intensity (counts)
2θ (°)
0
400
600
800
1,000
1,200
1,400
1,600
1,800
2,000
2,200
2,400
2,800
10
20
30
40

FIG. 23

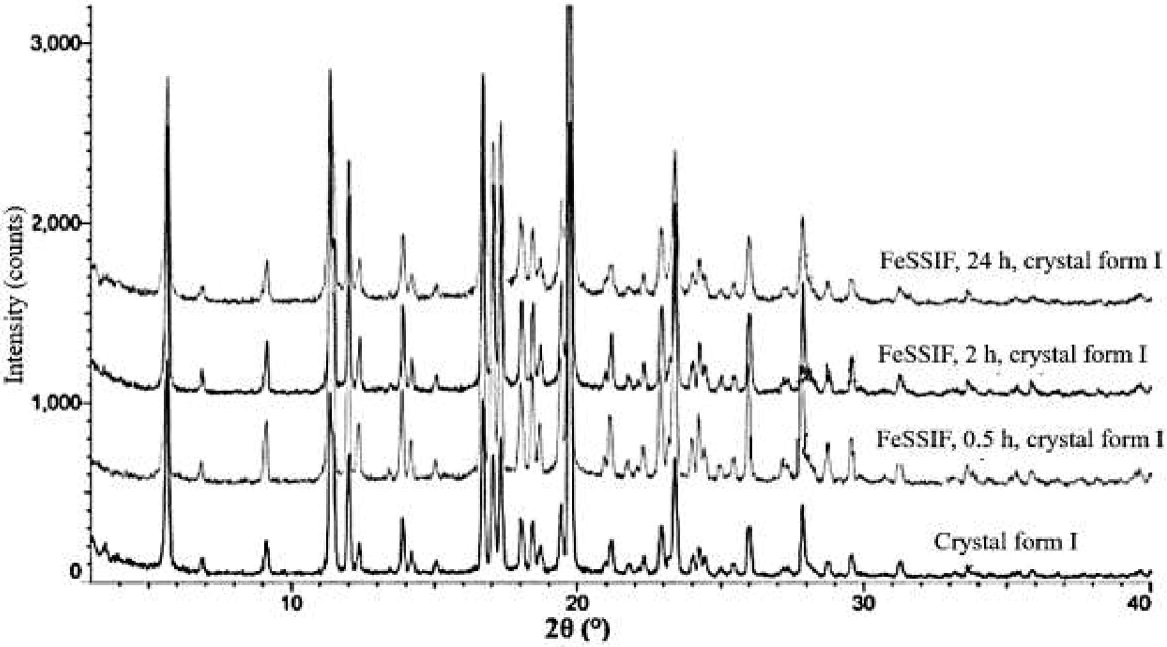
3,000
2,000
1,000
0
Intensity (counts)
10
20
30
40
2θ (°)
FeSSIF, 24 h, crystal form I
FeSSIF, 2 h, crystal form I
FeSSIF, 0.5 h, crystal form I
Crystal form I

FIG. 24

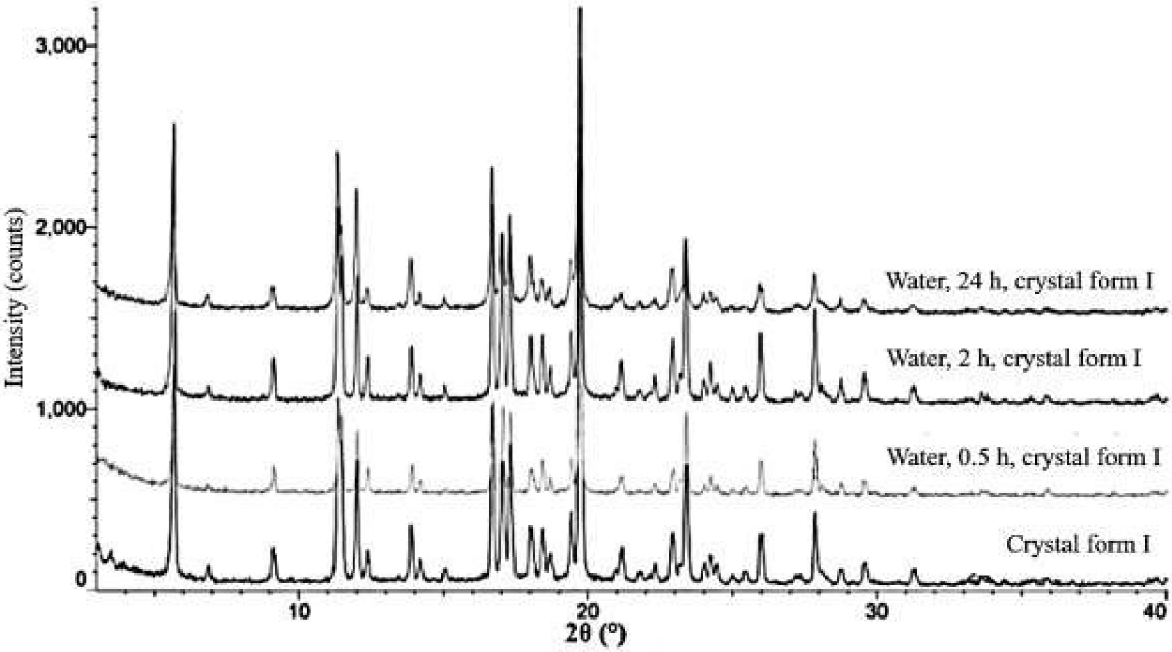
3,000
2,000
1,000
0
Intensity (counts)
10
20
30
40
2θ (°)
Water, 24 h, crystal form I
Water, 2 h, crystal form I
Water, 0.5 h, crystal form I
Crystal form I

FIG. 25

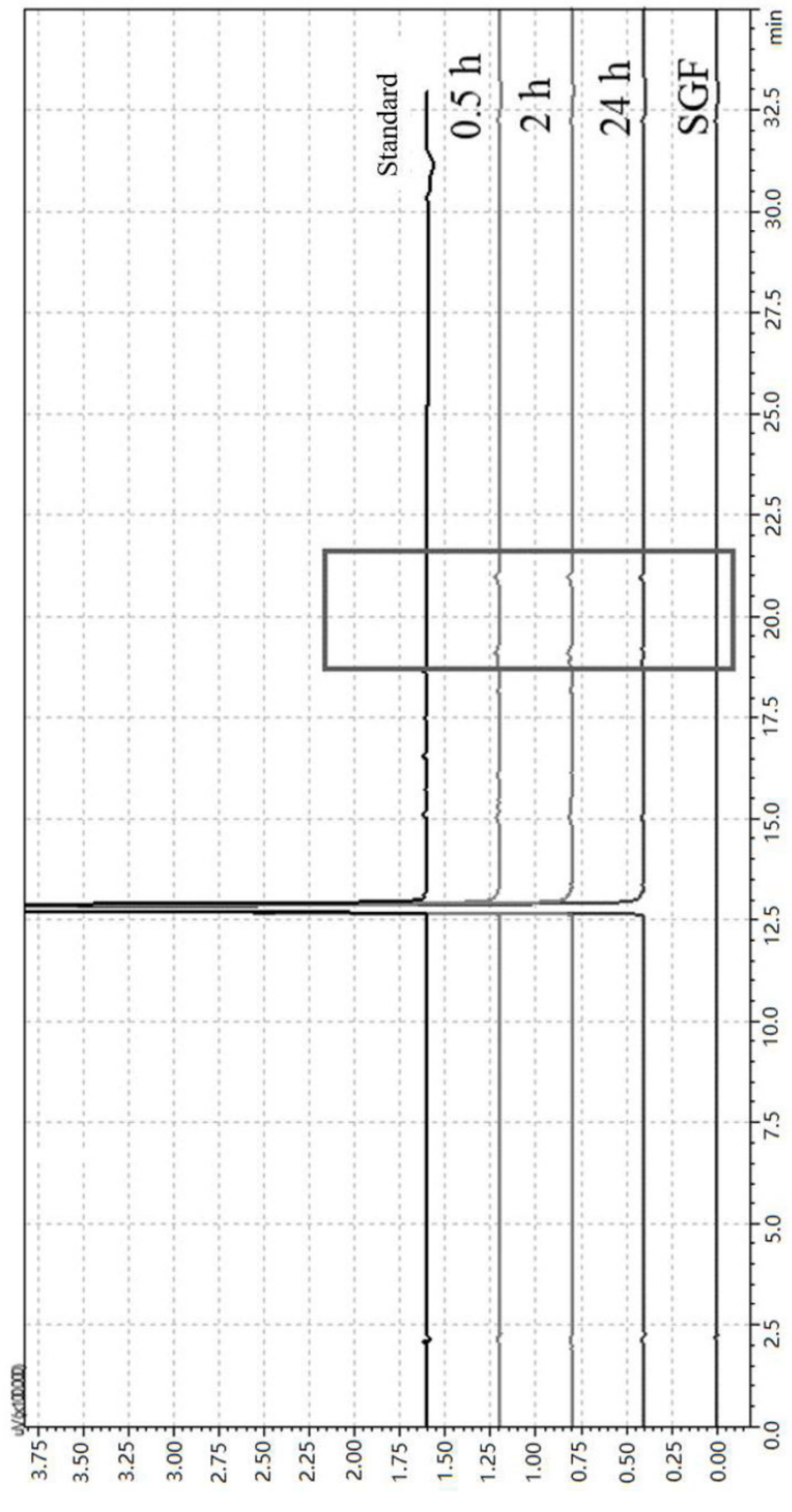
Standard
0.5 h
2 h
24 h
SGF
min
0.0
2.5
5.0
7.5
10.0
12.5
15.0
17.5
20.0
22.5
25.0
27.5
30.0
32.5
3.75
3.50
3.25
3.00
2.75
2.50
2.25
2.00
1.75
1.50
1.25
1.00
0.75
0.50
0.25
0.00

FIG. 26

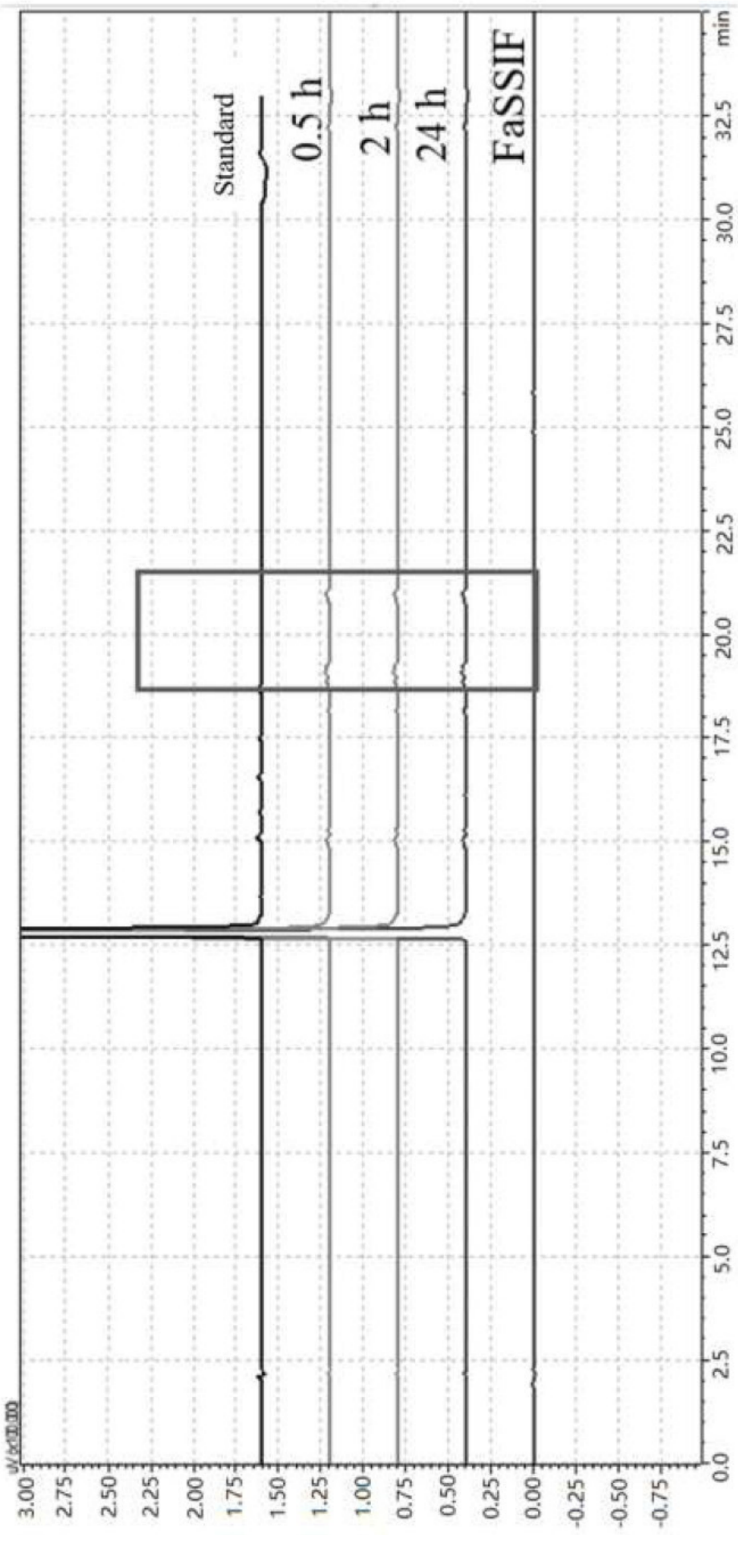
Standard
0.5 h
2 h
24 h
FaSSIF
min
3.00
2.75
2.50
2.25
2.00
1.75
1.50
1.25
1.00
0.75
0.50
0.25
0.00
-0.25
-0.50
-0.75
0.0
2.5
5.0
7.5
10.0
12.5
15.0
17.5
20.0
22.5
25.0
27.5
30.0
32.5

FIG. 27

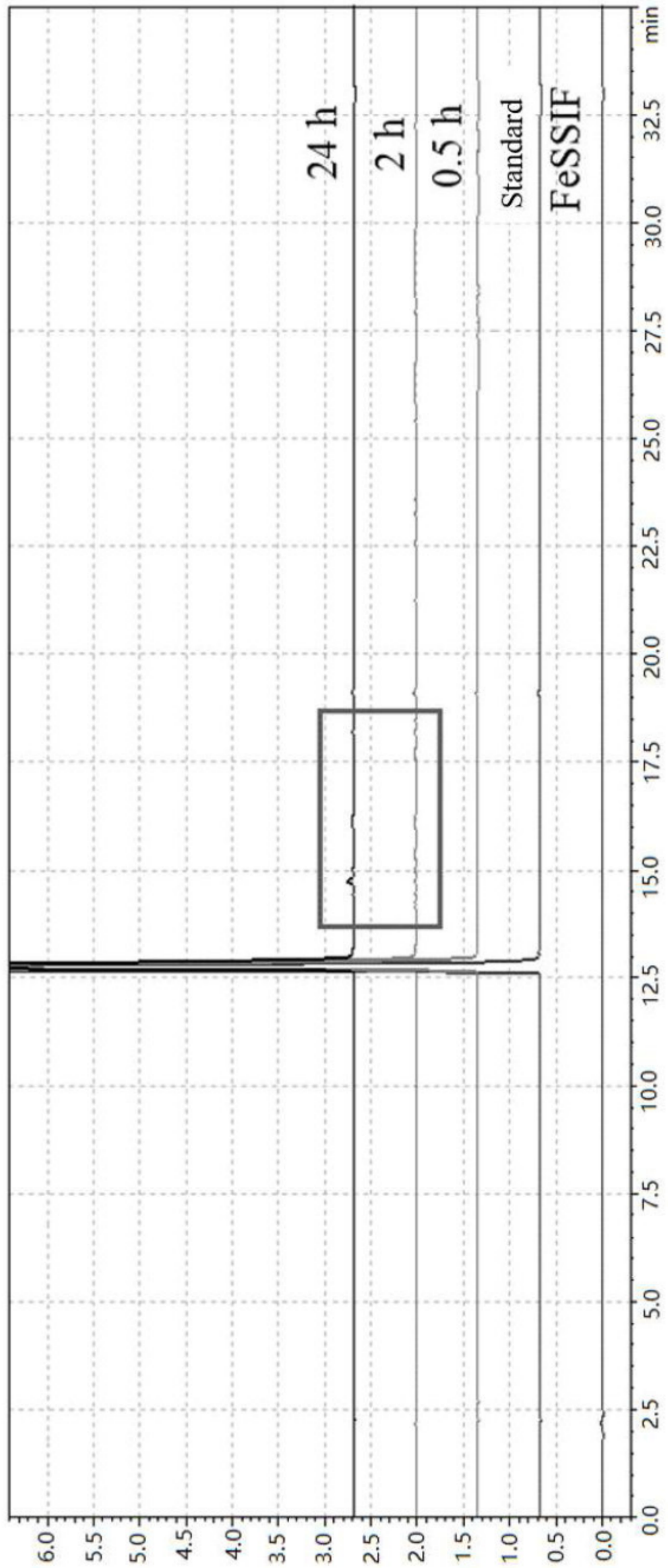
24 h
2 h
0.5 h
Standard
FeSSIF
min
0.0
2.5
5.0
7.5
10.0
12.5
15.0
17.5
20.0
22.5
25.0
27.5
30.0
32.5
6.0
5.5
5.0
4.5
4.0
3.5
3.0
2.5
2.0
1.5
1.0
0.5
0.0

FIG. 28

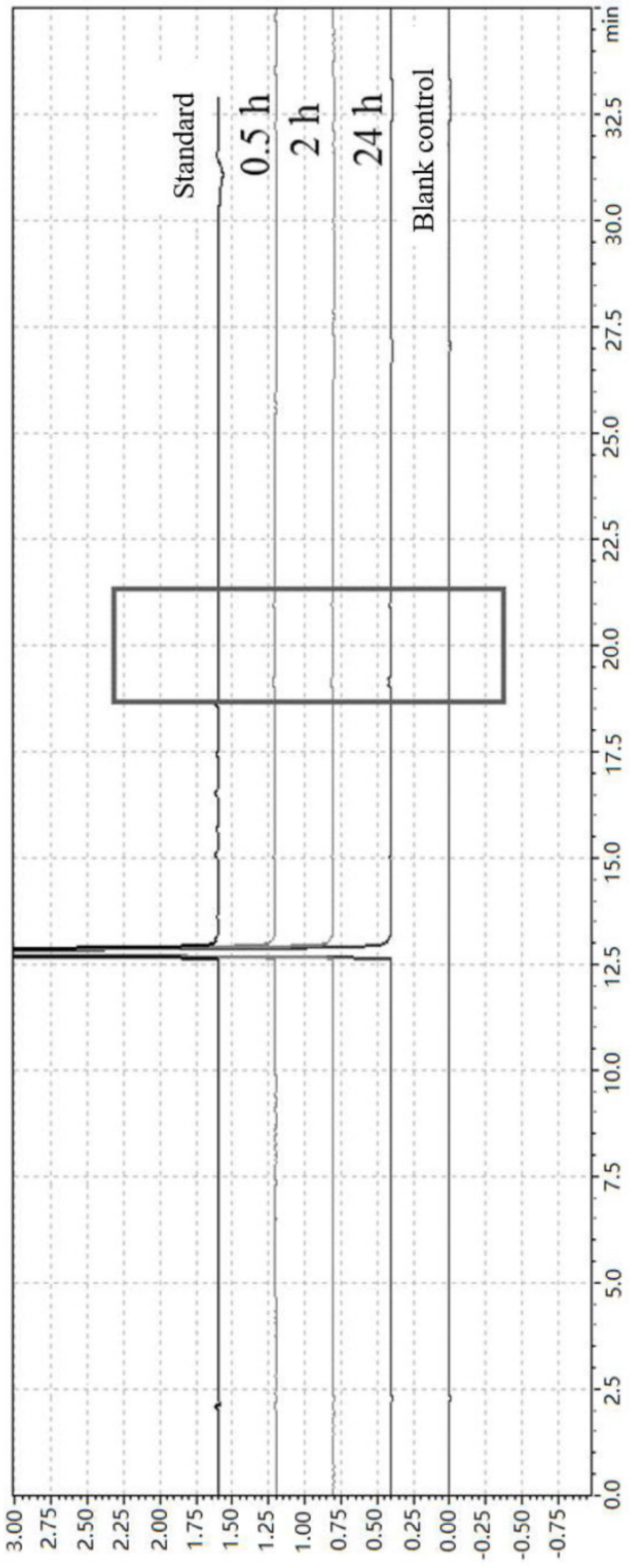
Standard
0.5 h
2 h
24 h
Blank control
min
0.0
2.5
5.0
7.5
10.0
12.5
15.0
17.5
20.0
22.5
25.0
27.5
30.0
32.5
3.00
2.75
2.50
2.25
2.00
1.75
1.50
1.25
1.00
0.75
0.50
0.25
0.00
-0.25
-0.50
-0.75

FIG. 29

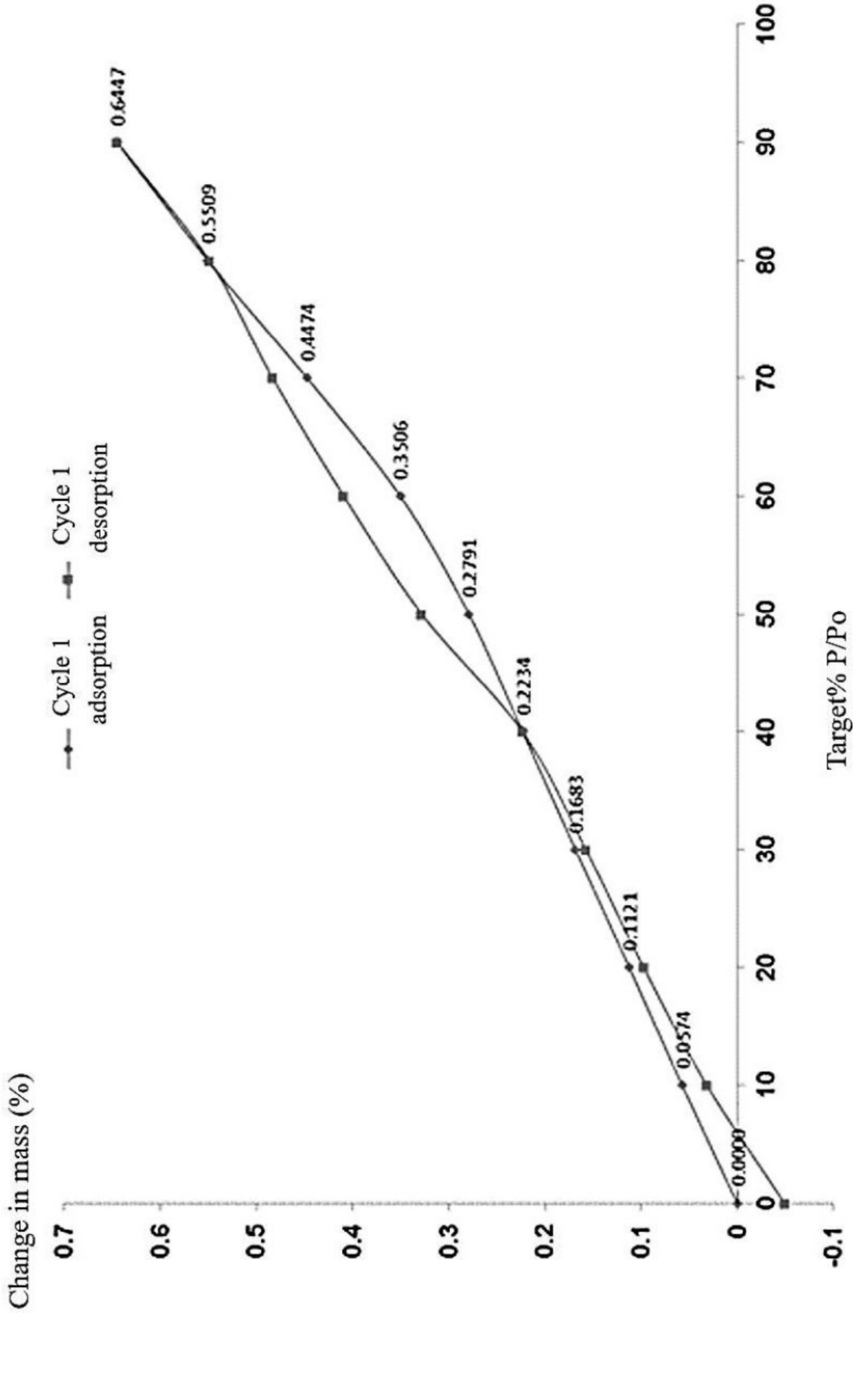
Change in mass (%)
Cycle 1 adsorption
Cycle 1 desorption
0.7
0.6
0.5
0.4
0.3
0.2
0.1
0
-0.1
0
10
20
30
40
50
60
70
80
90
100
0.0000
0.0574
0.1121
0.1683
0.2234
0.2791
0.3506
0.4474
0.5509
0.6447
Target% P/Po

FIG. 30

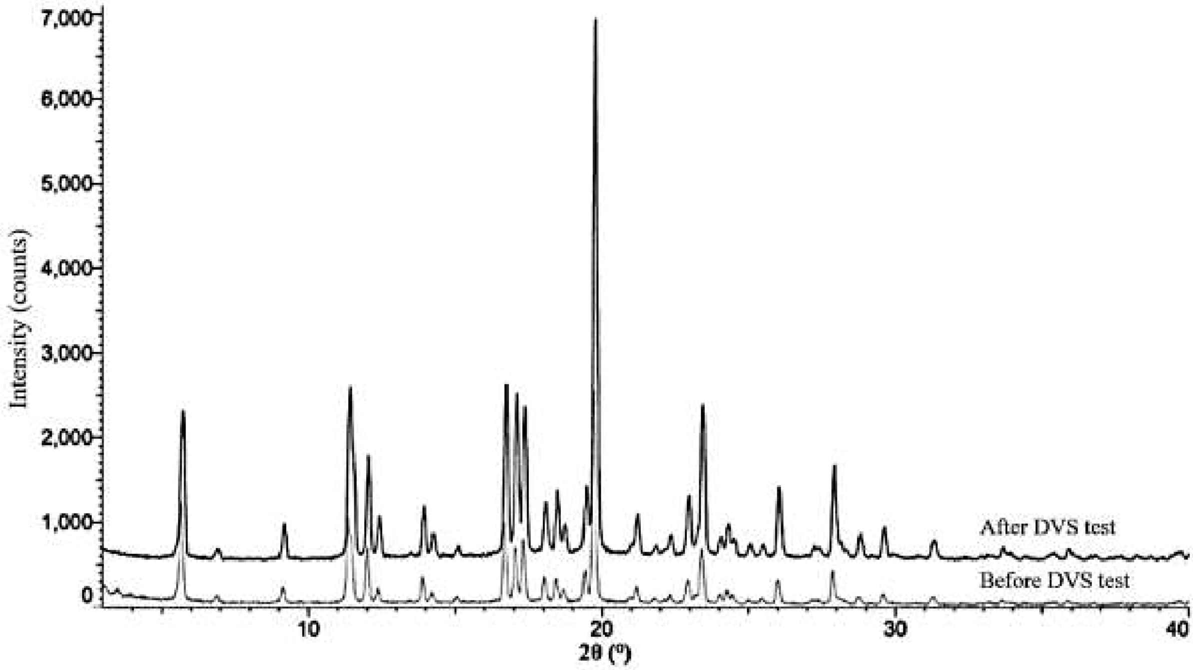
7,000
6,000
5,000
4,000
3,000
2,000
1,000
0
Intensity (counts)
After DVS test
Before DVS test
10
20
30
40
2θ (°)

FIG. 31

## INTERNATIONAL SEARCH REPORT

International application No.
**PCT/CN2024/084387**

**A. CLASSIFICATION OF SUBJECT MATTER**

C07D221/20(2006.01)i; C07D 471/10(2006.01)i; C07D209/54(2006.01)i; C07C69/734(2006.01)i; A61P7/00(2006.01)i; A61P9/00(2006.01)i; A61P37/02(2006.01)i; A61P25/28(2006.01)i; A61P11/00(2006.01)i; A61P13/00(2006.01)i; A61P13/12(2006.01)i; A61P27/02(2006.01)i; A61P29/00(2006.01)i; A61P19/02(2006.01)i; A61P17/06(2006.01)i; A61P21/04(2006.01)i; A61P31/14(2006.01)i; A61K31/438(2006.01)i; A61K 31/395(2006.01)i

According to International Patent Classification (IPC) or to both national classification and IPC

**B. FIELDS SEARCHED**

Minimum documentation searched (classification system followed by classification symbols)

IPC: C07D,C07C,A61P,A61K

Documentation searched other than minimum documentation to the extent that such documents are included in the fields searched

Electronic data base consulted during the international search (name of data base and, where practicable, search terms used)

CNABS; CNTXT; DWPI; CNKI; ENTXT; ENTXTC; 超星读秀, CHAOXING DUXIU; ISI_Web of Science; STN-REG; STN-CAPLUS: 武汉朗来科技发展有限公司, 胡钦铨, 娄军, 王亮, 张轶涵, structural formula search, 补体因子, 晶体, 晶型, complement, crystal, form

**C. DOCUMENTS CONSIDERED TO BE RELEVANT**

| Category* | Citation of document, with indication, where appropriate, of the relevant passages | Relevant to claim No. |
|---|---|---|
| A | CN 105555766 A (NOVARTIS AG) 04 May 2016 (2016-05-04)<br>description, paragraphs 2737-2739 and 3432-3434 | 1-12 |
| A | CN 115362162 A (ACHILLION PHARMACEUTICALS, INC.) 18 November 2022 (2022-11-18)<br>entire document | 1-12 |
| A | AR 96961 A1 (NOVARTIS AG) 10 February 2016 (2016-02-10)<br>entire document | 1-12 |

☐ Further documents are listed in the continuation of Box C.    ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 May 2024** | **24 June 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/084387**

| Patent document cited in search report | | | Publication date (day/month/year) | Patent family member(s) | | | Publication date (day/month/year) |
|---|---|---|---|---|---|---|---|
| CN | 105555766 | A | 04 May 2016 | TW | 201546030 | A | 16 December 2015 |
| | | | | JP | 2016529238 | A | 23 September 2016 |
| | | | | MX | 2016000675 | A | 10 May 2016 |
| | | | | US | 2016145247 | A1 | 26 May 2016 |
| | | | | EP | 3022182 | A1 | 25 May 2016 |
| | | | | CA | 2917614 | A1 | 22 January 2015 |
| | | | | WO | 2015009977 | A1 | 22 January 2015 |
| | | | | AU | 2014290498 | A1 | 04 February 2016 |
| | | | | KR | 20160036568 | A | 04 April 2016 |
| | | | | IN | 201617003907 | A | 22 July 2016 |
| | | | | BR | 112016000971 | A2 | 25 July 2017 |
| CN | 115362162 | A | 18 November 2022 | WO | 2021168320 | A1 | 26 August 2021 |
| | | | | EP | 4107166 | A1 | 28 December 2022 |
| | | | | JP | 2023515073 | A | 12 April 2023 |
| AR | 96961 | A1 | 10 February 2016 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023103175276
- CN 2024102275411